(19) Europäisches Patentamt
European Patent Office
Office européen des brevets

(11) **EP 4 376 011 A1**

(12) **EUROPEAN PATENT APPLICATION**

(43) Date of publication:
**29.05.2024   Bulletin 2024/22**

(21) Application number: **22209238.9**

(22) Date of filing: **23.11.2022**

(51) International Patent Classification (IPC):
**G16C 20/30** *(2019.01)*       *G16C 20/70* *(2019.01)*

(52) Cooperative Patent Classification (CPC):
**G16C 20/30;** G06N 5/01; G06N 20/10; G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Qsar Lab Spolka z. o.o**
**80-172 Gdansk (PL)**

(72) Inventors:
• **Wyrzykowska, Ewelina**
**19-300 Elk (PL)**

• **Stepnik, Maciej**
**94-126 Lodz (PL)**
• **Wojciechowska, Alicja**
**06-300 Przasnysz (PL)**
• **Nimz, Kinga**
**83-200 Starogard Gdanski (PL)**
• **Gromelski, Maciej**
**82-300 Elblag (PL)**
• **Puzyn, Tomasz**
**80-438 Gdansk (PL)**

(74) Representative: **JD&P Patent Attorneys**
**Joanna Dargiewicz & Partners**
**ul. Mysliborska 93A/50**
**03-185 Warszawa (PL)**

(54) **IN SILICO METHODS FOR PREDICTION OF INCREASED EXPRESSION OF PROINFLAMMATORY CYTOKINES IL 6, AND TNF ALPHA IN MAMMALIAN CELL LINES IN VITRO WITH TITANIUM DIOXIDE NANOPARTICLES**

(57)    The invention relates to four *in silica* methods for prediction of expression of strong proinflammatory cytokines: Interleukin-6 (IL-6), and Tumor Necrosis Factor-$\alpha$ (TNF-$\alpha$) in mammalian cell lines treated *in vitro* with titanium dioxide (TiO$_2$) nanoparticles, based on Qualitative Structure-Activity Relationships modelling (QSAR).

EP 4 376 011 A1

**Description**

The invention relates to four *in silica* methods for prediction of expression of strong proinflammatory cytokines: Interleukin-6 (IL-6), and Tumor Necrosis Factor-$\alpha$ (TNF-$\alpha$) in mammalian cell lines treated *in vitro* with titanium dioxide (TiO$_2$) nanoparticles, based on Qualitative Structure-Activity Relationships modelling (QSAR). The provided methods are grouped into two approaches: 1) prediction of cytokine (IL-6 or TNF-$\alpha$) induction with positive/negative response for defined TiO$_2$ nanoparticles (approach A), and 2) prediction of 'no observed effect concentration' (NOEC) of a TiO$_2$ nanoform, which is the highest concentration potentially used in experiments which should not induce a statistically significant increase in the cytokine (IL-6 or TNF-$\alpha$) production; the predicted NOEC can be classified into three classes of concentration ranges (approach B).

Several *in vivo* studies have identified the tumourigenic activity of TiO$_2$ after inhalation exposure. Human epidemiological data are limited and do not show any significantly elevated risk of lung cancer in association with TiO$_2$ exposure. International Agency Research on Cancer (IARC) classified TiO$_2$ as "possibly carcinogenic to humans (group 2B)" in 2010, followed by National Institute for Occupational Safety & Health (NIOSH) in 2011 indicating concerns that ultrafine TiO$_2$ might be carcinogenic in occupational settings. From the available rodent studies, the mechanisms by which TiO$_2$ can induce lung tumours in rats after inhalation are considered to be via impaired clearance and persistent inflammation.

The proinflammatory environment can increase the risk of many different pathological changes in the organism, ranging from hypersensitivity reactions, autoimmunity, chronic inflammation, fibrosis, to cancer development. Cancer cells can be stimulated by different bioactive molecules, including cytokines, growth factors, and chemokines that sustain cell proliferative rate, inhibit apoptosis, and stimulate angiogenesis, and extracellular matrix modifying enzymes, such as metalloproteinases, which promote the epithelial-mesenchymal transition (EMT). Among the most potent proinflammatory cytokines are IL-6 and TNF-$\alpha$.

IL-6 is produced by a number of cell types and exerts pleiotropic effects. Neutrophils and monocytes/macrophages produce and respond to IL-6, which can result in the amplification of inflammation and a switch from an acute to a chronic inflammatory state. IL-6 is involved in mediating the activation of T and B cells, which are the main drivers of adaptive immune responses and key cells in the pathogenesis of many autoimmune diseases. It is also implicated in the pathogenesis of fibrotic diseases, including lung and liver fibrosis.

The role of TNF-$\alpha$ in inflammation process is linked with induction of vasodilatation and edema formation, leukocyte adhesion to epithelium, through expression of adhesion molecules. It also regulates blood coagulation, contributes to oxidative stress in sites of inflammation, indirectly induces fever. TNF-$\alpha$ is also thought to contribute to carcinogenesis, especially in the early stages, including angiogenesis and invasion.

Cytokine expression can be evaluated at the level of gene or protein expression. Cytokine proteins can be measured in sera and in cell culture supernatants by Enzyme-Linked Immunosorbent Assay (ELISA) or by Western blotting, using antibody pairs recognizing the specific cytokine, or by bioassays. Bioassays are based either on cellular proliferative responses to specific cytokines or on cytotoxicity. Gene expression can be monitored using reverse-transcriptase polymerase chain reaction (RT-PCR). This method is based on reverse transcription of mRNA and amplification to detectable levels of cDNA by PCR. Cytokine production can be analyzed by automatized methods at the single-cell level by flow cytometry, detecting cells labelled with fluorescent conjugated antibodies. There are many immunofluorescent staining-based platforms developed by different manufacturers allowing simultaneous quantitation of numerous cytokines in one sample, e.g. Milliplex™ Map (Millipore), Bio-Plex Systems (Bio-Rad), or BD CBA Human Inflammatory Cytokine Kit.

In pursuance of the Regulation (EC) No. 1907/2006 of the European Parliament and the Council from December 18, 2006, on the Registration, Evaluation, Authorization and Restriction of Chemicals (REACH), there is a requirement of a detailed chemical risk assessment of each manufactured or imported substance placed on the European market. However, according to the estimations of the Joint Research Centre of the European Commission (JRC), a need of experimental testing enlargement would cause the use of approx. 3.9 million vertebrate animals and substantial increase of costs and time for chemicals safety assessment. Facing this, a number of alternative methods to toxicity assessment was proposed to fulfil at least one of the 3R's principles proposed by Russell and Burch (The Principles of Humane Experimental Technique, 1959) - Replacement, Reduction and Refinement of animal testing. Therefore, next to the welfare of laboratory animals and ethical aspects, economic and practical reasons were considered. Among the recommended alternative approaches, *in silico* modeling was pointed out, especially Structure-Activity Relationships modelling, where Qualitative Structure-Activity Relationships (QSAR) is one of the methods.

Titanium dioxide nanoparticles are one of the most commonly manufactured nanomaterials worldwide. Due to diversified applications, TiO$_2$ nanoparticles are produced with diversified physicochemical characteristics e.g., size distribution, particles' shape and surface chemistry resulting from the type of coating. Consequently, these differences are responsible for the observed differentiation of their bioactivity, including toxic effects. According to the European Commission Regulation (EU, 2018/1881), nanoparticles of the same substance but diversified under physicochemical characteristics, are considered as different 'nanoforms' of the same substance. Thus, each $TiO_2$ nanoform ($TiO_2$ nanoparticles differing in size/shape/surface chemistry etc.) should be analysed separately due to possible different biological effects. However, due to the financial cost-ineffectiveness and ethically questionable *in vivo* experimental research for each nanoform, computational methods i.e., QSAR modelling, are well appreciated as an effective alternative. Therefore, a set of computational methods - QSAR models - is a basic goal of the invention, to provide reliable and effective methods for prediction of expression of proinflammatory cytokines IL-6 and TNF-$\alpha$ in mammalian cell lines treated with $TiO_2$ nanoforms.

The idea of QSAR modelling is based on the assumption that there are quantitative or qualitative relationships between the structure of a substance and the observed biological activity. In QSAR modelling, the structure and properties of investigated substances are encoded numerically by so-called descriptors, where similar values of descriptors are associated with the similarity between substances. Following this, the greater physicochemical similarity between nanoforms, the more similar biological effects (e.g., mutagenicity, genotoxicity, immunotoxicity, etc.) will be observed for these nanoforms. The detailed relationships between descriptors and the interesting biological effect or physicochemical property (the subject of analysis) are investigated with supervised machine learning methods. These methods include diversified algorithms, like k-Nearest Neighbours (k-NN), decision tree, random forests, supporting vector machines, etc., and allow for the development of regression and classification models, depending on the modelled quantity. In regression models, the predicted value is expressed with continuous values, while in classification cases, as categories (e.g., toxic, or non-toxic class). Through the identification of the correlation between the structural characteristic of substances with the observed biological effect, QSAR modelling allows to increase the understanding of the investigated biological mechanism. More importantly, the developed QSAR models can be used to predict the interesting effect for new, untested substances (e.g., $TiO_2$ nanoforms) based solely on a set of descriptors required in the model, instead of performing costly and time-consuming experimental testing.

In the provided methods - QSAR models - there are applied data extracted from the available open literature on IL-6 and TNF-a induction in mammalian cells after exposure to different $TiO_2$ nanoforms. The set of nanoforms descriptors are extended by employing additional information on each *in vitro* experiment such as: cell line origin (human, mouse, rat), cell type and organ of origin (e.g., macrophage from lung, epithelium from liver, microglia from brain), germ layers the cells originate from (endoderm, ectoderm and mesoderm), biological nature of the cells used (normal, cancer), and concentration of $TiO_2$ nanoparticles used in the experiment (only in approach A; in approach B cannot be used due to a natural consequence of the modelled parameter character). These supplemental descriptors enveloped in presented approaches allow to identify distinct sensitivity of exposed cell lines to $TiO_2$ nanoforms to induce expression of the proinflammatory cytokines. Therefore, QSAR models' predictions are strictly adjusted to the specificity of particular cell lines investigated by the user. Moreover, apart from the general approach of QSAR modelling, where the model calibration is limited by the number of samples in the training set (the ratio between number of samples to the number of descriptors in the model should be at least 5:1), in presented approaches, this rule may be omitted due to dimensionality reduction of original datasets (matrices that characterize both nanoforms and cell lines). The dimensionality reduction of matrices is performed through principal component analysis (PCA). Obtained principal components (PCs) retain information from original matrices in condensed form, being linear combinations of the original variables (descriptors). The contribution of each descriptor in the newly formed PCs is derived from so-called loadings (computed by calculating Pearson's correlation coefficient between each original variable and PCs). The original information retained by each PC may be quantified by calculating percentage of expressed variance. Each computed PC explains unique portion of variance and the expression of the information is the greatest for the first PC and decreases for subsequent PCs. As a consequence, the obtained PCs are mutually orthogonal (each PC explains unique portion of the information). After PCA transformation, each sample receives new coordinates in the theoretical space of PCs, so-called scores (instead of coordinates in theoretical space of original variables). However, as the PCA produces the same number of PCs as variables in the original matrices, the presented approaches A and B include a step of reducing the number of PCs in order to maximise information about the nanoforms and cell lines characteristics while complying with the abovementioned 5:1 rule. In presented approaches the cut-off threshold for the reduction of the PCs number is at least 50% of the summarized expressed variance. As a result of aforementioned pre-processing techniques, the conclusive QSAR modelling is based on the reduced number of PCs for nanoform and cell line characteristics (and 'Concentration' vector in method A-1, and A-2).

The invention are four methods described below:

    1. Qualitative structure-activity relationship (QSAR) method for prediction of cytokine IL-6 induction in mammalian cell lines treated with $TiO_2$ nanoparticles, that can be used as an equivalent of *in vitro* experimental measurement with ELISA, RT-PCR or other test assay dedicated to identification of cytokines expression, comprises of the following steps:

        a) providing a dataset of $TiO_2$ nanoform(s) and cell line(s) characteristics, as well as corresponding concentration(s) at which influence on the IL-6 induction was observed in *in vitro* experimental measurements and used as training and validation sets, Table A1_1 - A1_4;

b) providing dataset pre-processing through standardization, principal component analysis (PCA) and reduction of a number of principal components (PCs) for the separated matrices of nanoforms and cell lines characteristics, with subsequent reconstruction of the decision tree classifier with inputs obtained for training set: i) matrices of reduced number of PCs describing nanoforms and cell lines characteristics, and ii) vector of corresponding concentrations;

c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics and concentrations, through: i) adherence to a specific group with binary scale (1 - yes, or 0 - no), ii) a value of a specific feature within the indicated range; the characteristics should be expressed as follows:

- cell line origin:

  ◦ human [1 or 0]
  ◦ mouse [1 or 0]
  ◦ rat [1 or 0]

- cell type and organ of origin:

  ◦ epithelium lung [1 or 0]
  ◦ epithelium cervix [1 or 0]
  ◦ macrophage monocyte [1 or 0]
  ◦ epithelium liver [1 or 0]
  ◦ neuron brain [1 or 0]
  ◦ microglia brain [1 or 0]
  ◦ macrophage lung [1 or 0]
  ◦ macrophage bone marrow [1 or 0]
  ◦ endothelium umbilical vein [1 or 0]
  ◦ dendritic monocytes [1 or 0]

- germ layers the cells originate from:

  ◦ endoderm [1 or 0]
  ◦ ectoderm [1 or 0]
  ◦ mesoderm [1 or 0]

- biological nature of cell used:

  ◦ normal [1 or 0]
  ◦ cancer [1 or 0]

- shape:

  ◦ spherical [1 or 0]
  ◦ irregular [1 or 0]
  ◦ inconclusive [1 or 0]

- size:

  ◦ min - minimum particle size, quantitative factor, derived from microscopic TEM images: 4.0-53.0 [nm]
  ◦ mean - mean particle size, quantitative factor, derived from microscopic TEM images: 5.8-110.0 [nm]
  ◦ max - maximum particle size, quantitative factor, derived from microscopic TEM images: 5.8-167.0 [nm]

- crystal structure:

  ◦ anatase - quantitative factor defining fraction of anatase form in the material: 0.0-1.0

◦ rutile - quantitative factor defining fraction of rutile form in the material: 0.0-1.0

▪ surface chemistry - qualitative factor defining presence of a coating on nanomaterial surface, expressed in a binary scale i.e.: 1 - presence of coating, 0 - absence of coating;

wherein the tested $TiO_2$ nanoforms concentrations should be in range 0.08-300.00 [$\mu$g/mL];
d) application of the developed model in the form of decision tree classifier for newly defined samples and prediction of IL-6 induction as positive or negative response based on provided descriptors.

2. Qualitative structure-activity relationship (QSAR) method for prediction of cytokine TNF-$\alpha$ induction in mammalian cell lines treated with $TiO_2$ nanoparticles, that can be used as an equivalent of *in vitro* experimental measurement with ELISA, RT-PCR or other test assay dedicated to identification of cytokines expression, comprises of the following steps:

a) providing a dataset of $TiO_2$ nanoform(s) and cell line(s) characteristics, as well as corresponding concentration(s) at which the TNF-$\alpha$ induction was observed in *in vitro* experiments and used as training and validation sets, Table A2_1 - A2_4;
b) providing dataset pre-processing through standardization, principal component analysis (PCA) and reduction of a number of principal components (PCs) for the separated matrices of nanoforms and cell lines characteristics, with subsequent reconstruction of the k-Nearest Neighbours classifier with inputs obtained for training set: i) matrices of reduced number of PCs describing nanoforms and cell lines characteristics, and ii) vector of corresponding concentrations;
c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics, and concentrations, through: i) adherence to a specific group with binary scale (1 - yes, or 0 - no), ii) a value of a specific feature within the indicated range; the characteristics should be expressed as follows:

▪ cell line origin:

◦ human [1 or 0]
◦ mouse [1 or 0]
◦ rat [1 or 0]

▪ cell type and organ of origin:

◦ macrophage monocyte [1 or 0]
◦ epithelium lung [1 or 0]
◦ macrophage lung alveolar [1 or 0]
◦ macrophage bone marrow [1 or 0]

▪ germ layers the cells originate from:

◦ endoderm [1 or 0]
◦ mesoderm [1 or 0]

▪ biological nature of cell used:

◦ normal [1 or 0]
◦ cancer [1 or 0]

▪ shape:

◦ spherical [1 or 0]
◦ elongated [1 or 0]
◦ inconclusive [1 or 0]

▪ size:

     ◦ min - minimum particle size, quantitative factor, derived from microscopic TEM images: 5.0-100.0 [nm]
     ◦ mean - mean particle size, quantitative factor, derived from microscopic TEM images: 5.0-110.0 [nm]
     ◦ max - maximum particle size, quantitative factor, derived from microscopic TEM images: 5.0-167.0 [nm]

    ■ crystal structure:

     ◦ anatase - quantitative factor defining fraction of anatase form in the material: 0.0-1.0
     ◦ rutile - quantitative factor defining fraction of rutile form in the material: 0.0-1.0

    ■ surface chemistry - qualitative factor defining presence of a coating on nanomaterial surface, expressed in a binary scale, i.e.: 1 - presence of coating, 0 - absence of coating;

wherein the tested $TiO_2$ nanoforms concentrations should be in range 0.5-304.00 [$\mu$g/mL];

d) application of the developed model in the form of k-Nearest Neighbours classifier for newly defined samples and prediction of TNF-$\alpha$ induction as positive or negative response based on provided descriptors.

3. Qualitative structure-activity relationship (QSAR) method for prediction of cytokine IL-6 NOEC class for mammalian cell lines treated with $TiO_2$ nanoparticles, that can be used as an equivalent of *in vitro* experiments using ELISA, RT-PCR or other test assay dedicated to identification of cytokines expression, comprises of the following steps:

a) providing a dataset of $TiO_2$ nanoform(s) and cell line(s) characteristics, as well as corresponding 'no observed effect concentration' (NOEC) which is the highest concentration potentially used in experiments which should not induce a statistically significant increase in the cytokine IL-6 production, obtained in *in vitro* experiments and used as training and validation sets, Table B1_1 - B1_3, where NOEC is classified into three classes of concentration ranges;

b) providing dataset pre-processing through standardization, principal component analysis (PCA) and reduction of a number of principal components (PCs) for the separated matrices of nanoforms and cell lines characteristics, with subsequent reconstruction of the decision tree classifier with inputs obtained for training set: matrices of reduced number of PCs describing nanoforms and cell lines characteristics;

c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics through: i) adherence to a specific group with binary scale (1 - yes, or 0 - no), ii) a value of a specific feature within the indicated range; the characteristics should be expressed as follows:

    ■ cell line origin:

     ◦ human [1 or 0]
     ◦ mouse [1 or 0]
     ◦ rat [1 or 0]

    ■ cell type and organ of origin:

     ◦ epithelium lung [1 or 0]
     ◦ epithelium cervix [1 or 0]
     ◦ macrophage monocyte [1 or 0]
     ◦ epithelium liver [1 or 0]
     ◦ neuron brain [1 or 0]
     ◦ microglia brain [1 or 0]
     ◦ macrophage lung [1 or 0]
     ◦ macrophage bone marrow [1 or 0]
     ◦ endothelium umbilical vein [1 or 0]
     ◦ dendritic monocytes [1 or 0]

    ■ germ layers the cells originate from:

     ◦ endoderm [1 or 0]
     ◦ ectoderm [1 or 0]

- mesoderm [1 or 0]

  - biological nature of cell used:

    - normal [1 or 0]
    - cancer [1 or 0]

  - shape:

    - spherical [1 or 0]
    - irregular [1 or 0]
    - inconclusive [1 or 0]

  - size:

    - min - minimum particle size, quantitative factor, derived from microscopic TEM images: 4.0-53.0 [nm]
    - mean - mean particle size, quantitative factor, derived from microscopic TEM images: 5.0-110.0 [nm]
    - max - maximum particle size, quantitative factor, derived from microscopic TEM images: 5.0-167.0 [nm]

  - crystal structure:

    - anatase - quantitative factor defining proportion of anatase form in the material: 0.0-1.0
    - rutile - quantitative factor defining proprtion of rutile form in the material: 0.0-1.0

  - surface chemistry - qualitative factor defining presence of a coating on nanomaterial surface, expressed in a binary scale, i.e.: 1 - presence of coating, 0 - absence of coating;

d) application of the developed model in the form of decision tree classifier for newly defined samples prediction of IL-6 NOEC class i.e., the highest $TiO_2$ nanoform concentration which does not induce IL-6 in exposed mammalian cells based on provided descriptors.

4. Qualitative structure-activity relationship (QSAR) method for prediction of cytokine TNF-$\alpha$ NOEC class for mammalian cell lines treated with $TiO_2$ nanoparticles, that can be used as an equivalent of *in vitro* experimental measurement with ELISA, RT-PCR or other test assay dedicated to identification of cytokines expression, comprises of the following steps:

a) providing a dataset of $TiO_2$ nanoform(s) and cell line(s) characteristics, as well as corresponding 'no observed effect concentration' (NOEC) which is the highest concentration potentially used in experiments which should not induce a statistically significant increase in the cytokine TNF-$\alpha$ production, obtained in *in vitro* experiments and used as training and validation sets, Table B2_1 - B2_3, where NOEC is classified into three classes of concentration ranges;
b) providing dataset pre-processing through standardization, principal component analysis (PCA) and reduction of a number of principal components (PCs) for the separated matrices of nanoforms and cell lines characteristics, with subsequent reconstruction of the random forest classifier with inputs obtained for training set: matrices of reduced number of PCs describing nanoforms and cell lines characteristics;
c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics through: i) adherence to a specific group with binary scale (1 - yes, or 0 - no), ii) a value of a specific feature within the indicated range; the characteristics should be expressed as follows:

  - cell line origin:

    - human [1 or 0]
    - mouse [1 or 0]
    - rat [1 or 0]

  - cell type and organ of origin:

- ○ macrophage monocyte [1 or 0]
- ○ epithelium lung [1 or 0]
- ○ macrophage lung alveolar [1 or 0]
- ○ macrophage bone marrow [1 or 0]

- germ layers the cells originate from:

  - ○ endoderm [1 or 0]
  - ○ mesoderm [1 or 0]

- biological nature of cell used:

  - ○ normal [1 or 0]
  - ○ cancer [1 or 0]

- shape:

  - ○ spherical [1 or 0]
  - ○ elongated [1 or 0]
  - ○ inconclusive [1 or 0]

- size:

  - ○ min - minimum particle size, quantitative factor, derived from microscopic TEM images: 5.3-100.0 [nm]
  - ○ mean - mean particle size, quantitative factor, derived from microscopic TEM images: 6.0-110.0 [nm]
  - ○ max - maximum particle size, quantitative factor, derived from microscopic TEM images: 6.7-167.0 [nm]

- crystal structure:

  - ○ anatase - quantitative factor defining fraction of anatase form in the material: 0.0-1.0
  - ○ rutile - quantitative factor defining fraction of rutile form in the material: 0.0-1.0

- surface chemistry - qualitative factor defining presence of a coating on nanomaterial surface, expressed in a binary scale, i.e.: 1 - presence of coating, 0 - absence of coating;

d) application of the developed model in the form of random forest classifier for newly defined samples and prediction of TNF-$\alpha$ NOEC class, the highest TiO$_2$ nanoform concentration which does not induce TNF-$\alpha$ in exposed mammalian cells based on provided descriptors.

According to the invention, provided methods (QSAR models) enable to predict the biological responses - expression of proinflammatory cytokines IL-6, and TNF-$\alpha$ - in mammalian cell lines after exposure to TiO$_2$ nanoparticles based on characteristics of these cell lines and physicochemical properties of nanoforms called here as descriptors. Each QSAR model was developed and validated based on experimental data for a set TiO$_2$ nanoforms for expression of selected proinflammatory cytokines: IL-6, and TNF-$\alpha$. The experimental *in vitro* testing was performed for limited number of TiO$_2$ nanoforms according to the recommended assays for nanomaterials e.g., ELISA or RT-PCR. However, the identified relationships between cell lines and nanoforms characteristics with the observed expression of selected proinflammatory cytokines through the provided approaches (QSAR models), allow for computational prediction of cytokine expression for the selected cell lines when treated with new, untested TiO$_2$ nanoforms, rather than performing their experimental testing. The invention, therefore, is computer-assisted methods (QSAR models) for the estimated potential of new, untested TiO$_2$ nanoforms to induce expression of IL-6, and TNF-$\alpha$ in selected cell lines, where each method depends on specific cell line(s) used during the calibration of a given QSAR model. Predictions from the provided methods can be used as an equivalent to *in vitro* experimental testing with ELISA, RT-PCR or other test assay dedicated to identification of cytokines expression.

DETAILED EXPLANATION

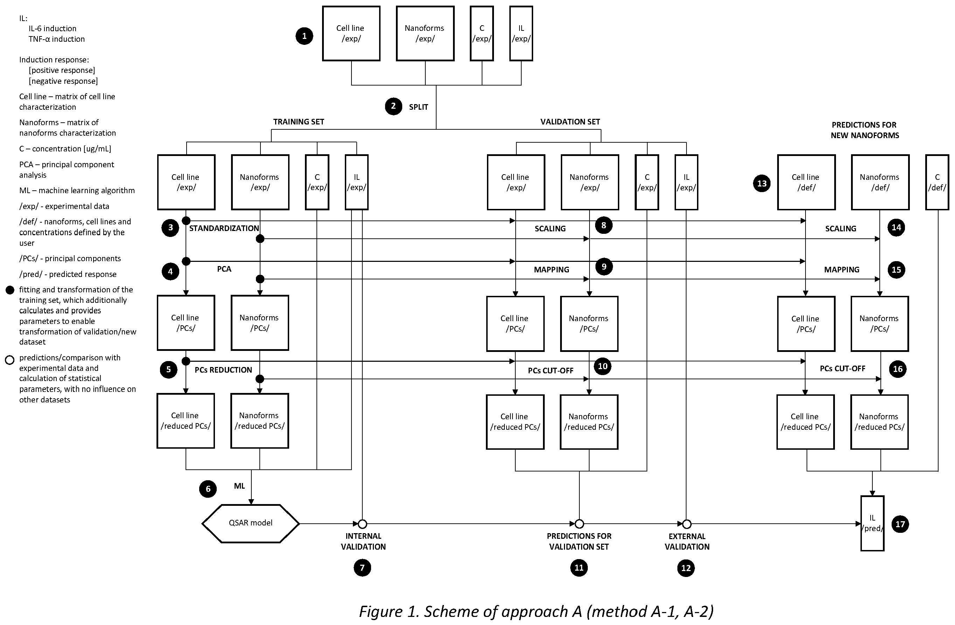

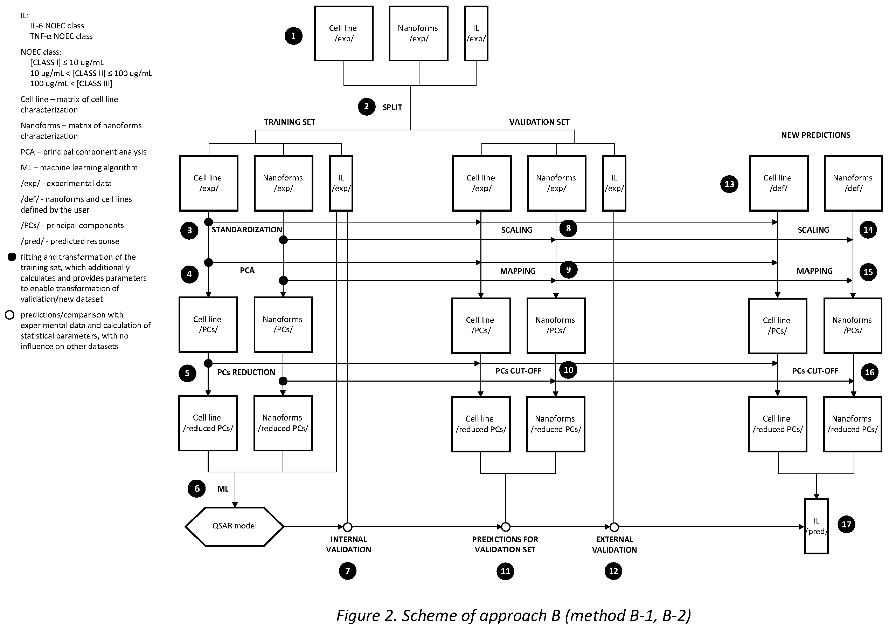

The invention involves four methods which are associated with two approaches (A and B) for prediction of expression of proinflammatory cytokines IL-6, and TNF-$\alpha$. Approach A allows for the prediction of induction of cytokines (positive/negative response) in mammalian cell lines treated with $TiO_2$ nanoparticles according to the general scheme presented on Figure 1. Within approach A, there are two methods: 1) method A-1 for prediction of IL-6 induction, and 2) method A-2 for prediction of TNF-$\alpha$ induction. Approach B allows for the prediction of the highest $TiO_2$ nanoform concentration which should not induce cytokines in exposed mammalian cells, expressed as NOEC values ('no observed effect concentration'). The response of NOEC prediction is expressed with one of three classes (ranges) of concentrations. General scheme of approach B is presented on Figure 2. Within approach B, there are two methods: 1) method B-1 for prediction IL-6 NOEC class, and 2) method B-2 for prediction TNF-$\alpha$ NOEC class.

Both approaches - A and B - are based on a separate pre-processing of matrices of cell lines and nanoforms characteristics. During the calibration of each QSAR model (methods: A-1, A-2, B-1, B-2), the initial (experimental) matrices of cell lines and nanoforms characteristics are transformed (standardized and subjected to principal component analysis), and these processes are mandatory to obtain predictions for new, untested $TiO_2$ nanoforms. The embodiment of the invention requires to recreate QSAR models (methods: A-1, A-2, B-1, B-2) according to the guidance provided for each method e.g., through applying the same training sets of cell lines, nanoforms, concentration (the last aspect applies only for methods A-1, A-2), observed response, as well as hyperparameters for machine learning methods. In further steps, scaling, mapping and predictions for the validation set are required. The correct recreation of a proper QSAR model is confirmed with statistical parameters for the training and validation set, which should be identical with these provided by Authors in this document. After this, the model can be applied for newly defined nanoform(s) and declared cell line(s). The applicability domain for each method is restricted to few aspects: range of descriptors' values for nanoforms, tested cell lines and used concentrations (the last aspect applies only for methods A-1, A-2).

According to the invention, based on provided QSAR models (methods: A-1, A-2, B-1, B-2), prediction of the expression of proinflammatory cytokines IL-6, and TNF-$\alpha$ in mammalian cell lines treated with new, untested $TiO_2$ nanoforms, requires information about nanoforms structure characteristics (related to size, shape, crystal structure and presence of coating), defining particular cell line and concentration (in case of methods A-1, A-2), and then reconstruction of a proper model according to the provided guidance. The credibility of the predictions is confirmed through the positive assessment of convergence of a new nanoform and defined experiment conditions i.e., cell lines (and concentration in the case of methods A-1, A-2) with the applicability domains of applied QSAR model.

Approach A - methods for prediction of cytokines induction

Method A-1 - prediction of IL-6 induction

Method A-1 employs a battery of common data curation and machine learning algorithms: standardization, dataset splitting based on Kennard-Stone algorithm, dimensionality reduction based on principal component analysis, model development based on decision tree classifier estimator. Said algorithms can be executed by means of any programming language and/or data analysis software.

The general scheme to recreate method A-1 of the invention is presented in Figure 1. The procedure is divided into 17 steps described below:

1. Datasets preparation

Method A-1 is based on an *in vitro* experimental data available for limited number of samples described with: i) matrix of cell lines characterization (Table A1_1), ii) matrix of nanoforms characterization (Table A1_2), iii) vector of nanoforms concentration (Table A1_3), and iv) observed level of IL-6 induction (Table A1_4). The observed responses for the available samples are as follows:

[positive response] 18 samples
[negative response] 15 samples

2. Splitting of the datasets

The available samples should be split into training and validation sets according to the Kennard-Stone algorithm, which allows for the creation of representative sets. The recreation of the A-1 model requires the same split, and it is available in Table A1_1 - A1_5. The training set is used for calibration and verification of the model robustness (internal validation). The validation set is used for verification of the model prediction ability (external validation). The ratio between number of samples in the training and validation set is 8:2 (26 training set: 7 validation set).

The number of samples with positive/negative responses in training set is as follows:

[positive response] 15 samples

[negative response] 11 samples

The number of samples with positive/negative responses in validation set is as follows:

[positive response] 3 samples
[negative response] 4 samples

3. [Training set] Standardization

In this step, the matrices of cell lines and nanoforms characteristics should be standardized separately according to the following equation (Eq.1):

$$ z_{AX} = \frac{x_{AX} - m_X}{s_X} \qquad (Eq.1) $$

Where: $z_{AX}$ - standardized value of 'X feature' for the 'A object'; $x_{AX}$ - original value of 'X feature' for the 'A object'; $m_X$ - mean value of 'X feature' for all objects in the training set; $s_X$ - standard deviation of 'X feature' for all objects in the training set.

4. [Training set] Principal Component Analysis

The standardized matrices should be used in principal component analysis algorithm in order to compute principal components (PCs) for further model development.

5. [Training set] PCs reduction

Due to the requirement of balanced proportion between the samples in training set and variables used in the model (abovementioned 5:1 rule), the number of PCs should be reduced to the minimum number of PCs expressing at least 50% of summarized variance. In this case, for the nanoforms characteristics and cell lines matrices the optimal number of PCs are two (PC1 34.5%, PC2 31.1%) and three (PC1 25.3%, PC2 17.6%, PC3 11.6%) respectively.

6. [Training set] Model calibration

Reduced number of PCs related to cell lines and nanoforms characteristics, corresponding concentration of nanoforms and observed response of cytokine induction are used for QSAR model development using machine learning method: decision tree classifier. Obtained scores values for samples in training set are reported in the Table A1_5. The hyperparameters for reproducing the model should be as follows:
*criterion: gini; splitter: best; max_depth: 4; min_samples_split: 2; min_samples_leaf: 1; min_weight_fraction_leaf: 0.0; max_features: None; random_state: 42; max_leaf_nodes: None; min_impurity_decrease: 0.0; class_weight: balanced; ccp_alpha: 0.0.*
At this step, a set of statistical parameters for training set should be as below:

Accuracy: 0.923
Precision avg. binary: 1.0
Precision avg. macro: 0.923
Precision avg. micro: 0.923
Precision avg. weighted: 0.935
Recall avg. binary: 0.867
Recall avg. macro: 0.933
Recall avg. micro: 0.923
Recall avg. weighted: 0.923
F1_score avg. binary: 0.929
F1_score avg. macro: 0.923
F1_score avg. micro: 0.923
F1_score avg. weighted: 0.924
MCC: 0.856

7. [Training set] Internal validation

The internal validation of the developed model should be verified by performing stratified k-fold cross-validation. The hyperparameter should be as follow:

*cv: 5.*

At this step, a set of statistical parameters for training set cross-validation should be as follow:

Accuracy: 0.680
Precision: 0.733
Recall: 0.733
F1: 0.733

8. [Validation set] Scaling

The validation set should be standardized by scaling the original values to the mean and standard deviation values calculated for the training set, according to the equation (Eq.2):

$$z_{BX} = \frac{x_{BX} - m_X}{s_X} \qquad \text{(Eq.2)}$$

Where: $z_{BX}$ - standardized value of 'X feature' for the 'B object' in the validation set; $x_{BX}$ - original value of 'X feature' for the 'B object' in the validation set; $m_X$ - mean value of 'X feature' for all objects in the training set; $s_X$ - standard deviation of 'X feature' for all objects in the training set.

9. [Validation set] Mapping

In this step, the standardized validation sets should be mapped (transformed) into principal components retaining all parameters from the PCA algorithm performed on the training set.

10. [Validation set] PCs cut-off

The selected number PCs for the validation set should be the same as in step 5 for each matrix: PC1 and PC2 for the nanoform characteristics matrix and PC1-PC3 for the cell lines matrix. Obtained scores values for samples in validation set are reported in the Table A1_5.

11. [Validation set] Predictions

In this step the developed model is used to predict biological response for samples in the validation set.

12. [Validation set] External validation

Predicted values from step 11 for each sample should be compared with the experimental responses to calculate validation statistical parameters.

The expected statistic values for the validation set should be as follow:

Accuracy: 0.857
Precision avg. binary: 1.000
Precision avg. macro: 0.900
Precision avg. micro: 0.857
Precision avg. weighted: 0.886
Recall avg. binary: 0.667
Recall avg. macro: 0.833
Recall avg. micro: 0.857
Recall avg. weighted: 0.857
F1_score avg. binary: 0.800
F1_score avg. macro: 0.844
F1_score avg. micro: 0.857

F1_score avg. weighted: 0.851
MCC: 0.730

13. [New predictions]

Defining nanoform, cell line and concentration This step requires to define input parameters for the new prediction (nanoform characteristics, cell line characteristics and concentration). In order to ensure high reliability of predictions, the provided values of input parameters should be within the ranges of defined applicability domain defined below. The order of input parameters should be the same as in the training set.

Cell lines and nanoforms should be characterized by belonging to each of the specific groups listed below. Characteristics are expressed in a binary scale, i.e.: 1 - the cell line/nanoform belongs to the group, 0 - the cell line/nanoform does not belong to the group.

- cell line origin:

  - human [1 or 0]
  - mouse [1 or 0]
  - rat [1 or 0]

- cell type and organ of origin:

  - epithelium lung [1 or 0]
  - epithelium cervix [1 or 0]
  - macrophage monocyte [1 or 0]
  - epithelium liver [1 or 0]
  - neuron brain [1 or 0]
  - microglia brain [1 or 0]
  - macrophage lung [1 or 0]
  - macrophage bone marrow [1 or 0]
  - endothelium umbilical vein [1 or 0]
  - dendritic monocytes [1 or 0]

- germ layers the cells originate from:

  - endoderm [1 or 0]
  - ectoderm [1 or 0]
  - mesoderm [1 or 0]

- biological nature of cell used:

  - normal [1 or 0]
  - cancer [1 or 0]

Nanoforms should be characterized in terms of the following features and belong within the indicated value ranges:

- shape:

  - spherical [1 or 0]
  - irregular [1 or 0]
  - inconclusive [1 or 0]

- size:

  - min - minimum particle size, quantitative factor, derived from microscopic TEM images: 4.0-53.0 [nm]
  - mean - mean particle size, quantitative factor, derived from microscopic TEM images: 5.8-110.0 [nm]
  - max - maximum particle size, quantitative factor, derived from microscopic TEM images: 5.8-167.0 [nm]

- crystal structure:

  ∘ anatase - quantitative factor defining fraction of anatase form in the material: 0.0-1.0
  ∘ rutile - quantitative factor defining fraction of rutile form in the material: 0.0-1.0

- surface chemistry - qualitative factor defining presence of a coating on nanomaterial surface, expressed in a binary scale i.e.: 1 - presence of coating, 0 - absence of coating.

Concentrations of nanoforms for new predictions should be within the ranges: 0.08-300.00 [μg/mL].

14. [New predictions] Scaling

The input parameters should be standardized by scaling the original values to the mean and standard deviation values calculated for the training set, according to the equation (Eq.3):

$$ z_{CX} = \frac{x_{CX} - m_X}{s_X} \qquad (Eq.3) $$

Where: $z_{CX}$ - standardized value of 'X feature' for the input 'C sample'; $x_{BX}$ - original value of 'X feature' for the input 'C sample'; $m_X$ - mean value of 'X feature' for all objects in the training set; $s_X$ - standard deviation of 'X feature' for all objects in the training set.

15. [New predictions] Mapping

In this step, the standardized input samples should be mapped (transformed) into principal components retaining all parameters from the PCA algorithm performed on the training set.

16. [New predictions] PCs cut-off

The selected number PCs for the input samples should be the same as in step 5 for each matrix: PC1 and PC2 for the nanoform characteristics matrix and PC1-PC3 for the cell lines matrix.

17. [New predictions] Predictions

In this step the developed model is used to predict biological response for the input samples - new, untested nanoform(s), defined cell line(s) and concentration(s). As a result, a positive or negative response is assigned to the defined samples.

Method A-2 - prediction of TNF-α induction

Method A-2 employs a battery of common data curation and machine learning algorithms: standardization, dataset splitting based on Kennard-Stone algorithm, dimensionality reduction based on principal component analysis, model development based on k-Nearest Neighbors classifier estimator. Said algorithms can be executed by means of any programming language and/or data analysis software.
The general scheme to recreate method A-2 of the invention is presented in Figure 1. The procedure is divided into 17 steps described below:

1. Datasets preparation

Method A-2 is based on an *in vitro* experimental data available for limited number of samples described with: i) matrix of cell lines characterization (Table A2_1), ii) matrix of nanoforms characterization (Table A2_2), iii) vector of nanoforms concentration (Table A2_3), and iv) observed level of TNF-α induction (Table A2_4). The observed responses for the available samples are as follows:

[positive response] 24 samples
[negative response] 8 samples

2. Splitting of the datasets

The available samples should be split into training and validation sets according to the Kennard-Stone algorithm, which allows for the creation of representative sets. The recreation of the A-2 model requires the same split, and it is available in Table A2_1 - A2_5. The training set is used for calibration and verification of the model robustness (internal validation). The validation set is used for verification of the model prediction ability (external validation). The ratio between number of samples in the training and validation set is 8:2 (25 training set: 7 validation set).
The number of samples with positive/negative responses in training set is as follows:

[positive response] 19 samples
[negative response] 6 samples

The number of samples with positive/negative responses in validation set is as follows:

[positive response] 5 samples
[negative response] 2 samples

3. [Training set] Standardization

In this step, the matrices of cell lines and nanoforms characteristics should be standardized separately according to the equation (Eq.1):

$$z_{AX} = \frac{x_{AX} - m_X}{s_X} \qquad \text{(Eq.1)}$$

Where: $z_{AX}$ - standardized value of 'X feature' for the 'A object'; $x_{AX}$ - original value of 'X feature' for the 'A object'; $m_X$ - mean value of 'X feature' for all objects in the training set; $s_X$ - standard deviation of 'X feature' for all objects in the training set.

4. [Training set] Principal Component Analysis

The standardized matrices should be used in principal component analysis algorithm in order to compute principal components (PCs) for further model development.

5. [Training set] PCs reduction

Due to the requirement of balanced proportion between the samples in training set and variables used in the model (abovementioned 5:1 rule), the number of PCs should be reduced to the minimum number of PCs expressing at least 50% of summarized variance. In this case, for the nanoforms characteristics and cell lines matrices the optimal number of PCs are two (PC1 37.1%, PC2 28.7%) and two (PC1 46.6%, PC2 29.0%) respectively.

6. [Training set] Model calibration

Reduced number of PCs related to cell lines and nanoforms characteristics, corresponding concentration of nanoforms and observed response of cytokine induction are used for QSAR model development using machine learning method: k-Nearest Neighbors classifier. Obtained scores values for samples in training set are reported in the Table A2_5. The hyperparameters for reproducing the model should be as follows:
*n_neighbours: 3; weights: uniform; algorithm: auto; leaf_size: 30; p: 2; metric: minkowski; metric_params: None; n_jobs: None.*
At this step, a set of statistical parameters for training set should be as below:

Accuracy: 0.920
Precision avg. binary: 0.905
Precision avg. macro: 0.952
Precision avg. micro: 0.920
Precision avg. weighted: 0.928
Recall avg. binary: 1.000

Recall avg. macro: 0.833
Recall avg. micro: 0.920
Recall avg. weighted: 0.920
F1_score avg. binary: 0.950
F1_score avg. macro: 0.875
F1_score avg. micro: 0.920
F1_score avg. weighted: 0.914
MCC: 0.777

7. [Training set] Internal validation

The internal validation of the developed model should be verified by performing stratified k-fold cross-validation. The hyperparameter should be as follow:
*cv: 5.*

At this step, a set of statistical parameters for training set cross-validation should be as follow:

Accuracy: 0.720
Precision: 0.750
Recall: 0.950
F1 score: 0.833

8. [Validation set] Scaling

The validation set should be standardized by scaling the original values to the mean and standard deviation values calculated for the training set, according to the equation (Eq.2):

$$z_{BX} = \frac{x_{BX} - m_X}{s_X} \qquad \text{(Eq.2)}$$

Where: $z_{BX}$ - standardized value of 'X feature' for the 'B object' in the validation set; $x_{BX}$ - original value of 'X feature' for the 'B object' in the validation set; $m_X$ - mean value of 'X feature' for all objects in the training set; $s_X$ - standard deviation of 'X feature' for all objects in the training set.

9. [Validation set] Mapping

In this step, the standardized validation sets should be mapped (transformed) into principal components retaining all parameters from the PCA algorithm performed on the training set.

10. [Validation set] PCs cut-off

The selected number PCs for the validation set should be the same as in step 5 for each matrix: PC1 and PC2 for the nanoform characteristics matrix; and PC1 and PC2 for the cell lines matrix. Obtained scores values for samples in validation set are reported in the Table A2_5.

11. [Validation set] Predictions

In this step the developed model is used to predict biological response for samples in the validation set.

12. [Validation set] External validation

Predicted values from step 11 for each sample should be compared with the experimental responses to calculate validation statistical parameters.
The expected statistic values for the validation set should be as follow:

Accuracy: 0.857
Precision avg. binary: 0.833
Precision avg. macro: 0.917

Precision avg. micro: 0.857
Precision avg. weighted: 0.881
Recall avg. binary: 1.000
Recall avg. macro: 0.750
Recall avg. micro: 0.857
Recall avg. weighted: 0.857
F1_score avg. binary: 0.909
F1_score avg. macro: 0.788
F1_score avg. micro: 0.857
F1_score avg. weighted: 0.840
MCC: 0.645

13. [New predictions] Defining nanoform, cell line and concentration

This step requires to define input parameters for the new prediction (nanoform characteristics, cell line characteristics and concentration). In order to ensure high reliability of predictions, the provided values of input parameters should be within the ranges of defined applicability domain defined below. The order of input parameters should be the same as in the training set.

Cell lines and nanoforms should be characterized by belonging to each of the specific groups listed below. Characteristics are expressed in a binary scale, i.e.: 1 - the cell line/nanoform belongs to the group, 0 - the cell line/nanoform does not belong to the group.

- cell line origin:

  ○ human [1 or 0]
  ○ mouse [1 or 0]
  ○ rat [1 or 0]

- cell type and organ of origin:

  ○ macrophage monocyte [1 or 0]
  ○ epithelium lung [1 or 0]
  ○ macrophage lung alveolar [1 or 0]
  ○ macrophage bone marrow [1 or 0]

- germ layers the cells originate from:

  ○ endoderm [1 or 0]
  ○ mesoderm [1 or 0]

- biological nature of cell used:

  ○ normal [1 or 0]
  ○ cancer [1 or 0]

Nanoforms should be characterized in terms of the following features and belong within the indicated value ranges:

- shape:

  ○ spherical [1 or 0]
  ○ elongated [1 or 0]
  ○ inconclusive [1 or 0]

- size:

  ○ min - minimum particle size, quantitative factor, derived from microscopic TEM images: 5.0-100.0 [nm]
  ○ mean - mean particle size, quantitative factor, derived from microscopic TEM images: 5.0-110.0 [nm]

◦ max - maximum particle size, quantitative factor, derived from microscopic TEM images: 5.0-167.0 [nm]

▪ crystal structure:

◦ anatase - quantitative factor defining proportion of anatase form in the material: 0.0-1.0
◦ rutile - quantitative factor defining proprtion of rutile form in the material: 0.0-1.0

▪ surface chemistry - qualitative factor defining presence of a coating on nanomaterial surface, expressed in a binary scale i.e.: 1 - presence of coating, 0 - absence of coating.

Concentrations of nanoforms for new predictions should be within the ranges:
0.5-304.00 [$\mu$g/mL].

14. [New predictions] Scaling

The input parameters should be standardized by scaling the original values to the mean and standard deviation values calculated for the training set, according to the equation (Eq.3):

$$z_{CX} = \frac{x_{CX} - m_X}{s_X} \qquad\qquad \text{(Eq.3)}$$

Where: $z_{CX}$ - standardized value of 'X feature' for the input 'C sample'; $x_{BX}$ - original value of 'X feature' for the input 'C sample'; $m_X$ - mean value of 'X feature' for all objects in the training set; $s_X$ - standard deviation of 'X feature' for all objects in the training set.

15. [New predictions] Mapping

In this step, the standardized input samples should be mapped (transformed) into principal components retaining all parameters from the PCA algorithm performed on the training set.

16. [New predictions] PCs cut-off

The selected number PCs for the input samples should be the same as in step 5 for each matrix: PC1 and PC2 for the nanoform characteristics matrix, and PC1 and PC2 for the cell lines matrix.

17. [New predictions] Predictions

In this step the developed model is used to predict biological response for the input samples - new, untested nanoform(s), defined cell line(s) and concentration(s). As a result, a positive or negative response is assigned to the defined samples.

Approach B - methods for prediction cytokines NOEC class

Method B-1- prediction of IL-6 NOEC class

Method B-1 employs a battery of common data curation and machine learning algorithms: standardization, dataset splitting based on Kennard-Stone algorithm, dimensionality reduction based on principal component analysis, model development based on decision tree classifier estimator. Said algorithms can be executed by means of any programming language and/or data analysis software.

The general scheme to recreate method B-1 of the invention is presented in Figure 2. The procedure is divided into 17 steps described below:

1. Datasets preparation

Method B-1 is based on an *in vitro* experimental data available for limited number of samples described with: i) matrix of cell lines characterization (Table B1_1), ii) matrix of nanoforms characterization (Table B1_2), and iii) the highest observed TiO$_2$ nanoform concentration which does not induce IL-6 in exposed mammalian cells, expressed as NOEC values ('no observed effect concentration') (Table B1_3). The observed NOEC is classified into three classes of concentration ranges:

[CLASS I] $\leq$ 10 $\mu$g/mL (11 samples)
10 $\mu$g/mL < [CLASS II] $\leq$ 100 $\mu$g/mL (12 samples)
100 $\mu$g/mL < [CLASS III] (7 samples)

2. Splitting of the datasets

The available samples should be split into training and validation sets according to the Kennard-Stone algorithm, which allows for the creation of representative sets. However, due to unbalanced classes of samples in the obtained sets i.e., high number of samples CLASS III and low number of samples CLASS II in validation set, the calibration of a model was performed on a modified training set. A randomly chosen single sample CLASS III (ID 23) was excluded from the validation set and included in training set, while sample CLASS II (ID 2) was moved from the training to validation set. The recreation of the B-1 model requires the same split, and it is available in Table B1_1 - B1_4. The training set is used for calibration and verification of the model robustness (internal validation). The validation set is used for verification of the model prediction ability (external validation). The ratio between number of samples in the training and validation set is 8:2 (24 training set: 6 validation set).
The number of classes in training set is as follows:

[CLASS I] 9 samples
[CLASS II] 10 samples
[CLASS III] 5 samples

The number of classes in validation set is as follows:

[CLASS I] 2 samples
[CLASS II] 2 sample
[CLASS III] 2 samples

3. [Training set] Standardization

In this step, the matrices of cell lines and nanoforms characteristics should be standardized separately according to the following equation (Eq.1):

$$z_{AX} = \frac{x_{AX} - m_X}{s_X} \qquad\qquad (Eq.1)$$

Where: $z_{AX}$ - standardized value of 'X feature' for the 'A object'; $x_{AX}$ - original value of 'X feature' for the 'A object'; $m_X$ - mean value of 'X feature' for all objects in the training set; $s_X$ - standard deviation of 'X feature' for all objects in the training set.

4. [Training set] Principal Component Analysis

The standardized matrices should be used in principal component analysis algorithm in order to compute principal components (PCs) for further model development.

5. [Training set] PCs reduction

Due to the requirement of balanced proportion between the samples in training set and variables used in the model (abovementioned 5:1 rule), the number of PCs should be reduced to the minimum number of PCs expressing at least 50% of summarized variance. In this case, for the nanoforms characteristics and cell lines matrices the optimal number of PCs are two (PC1 34.9%, PC2 29.9%) and three (PC1 25.8%, PC2 17.8%, PC3 11.0%) respectively.

6. [Training set] Model calibration

Reduced number of PCs related to cell lines and nanoforms characteristics, and observed NOEC class are used for QSAR model development using machine learning method: decision tree classifier. Obtained scores values for samples in training set are reported in the Table B1_4. The hyperparameters for reproducing the model should be as follows:

*criterion: gini; splitter: best; max_depth: 6; min_samples_split: 2;* **min_samples_leaf: 1; min_weight_fraction_leaf:** *0.0;* **max_features:** *None;* **random_state:** *42;* **max_leaf_nodes:** *None;* **min impurity_decrease:** *0.0;* **class_weight:** **balanced; ccp_alpha:** *0.0.*

At this step, a set of statistical parameters for training set should be as below:

Accuracy: 0.958
Precision avg. macro: 0.944
Precision avg. micro: 0.958
Precision avg. weighted: 0.965
Recall avg. macro: 0.963
Recall avg. micro: 0.958
Recall avg. weighted: 0.958
F1_score avg. macro: 0.950
F1_score avg. micro: 0.958
F1_score avg. weighted: 0.959
MCC: 0.938

7. [Training set] Internal validation

The internal validation of the developed model should be verified by performing stratified k-fold cross-validation. The hyperparameter should be as follow:

*cv: 5*

At this step, the statistical parameter for training set cross-validation should be as follow:

Accuracy: 0.58

8. [Validation set] Scaling

The validation set should be standardized by scaling the original values to the mean and standard deviation values calculated for the training set, according to the equation (Eq.2):

$$z_{BX} = \frac{x_{BX} - m_X}{s_X} \qquad \text{(Eq.2)}$$

Where: $z_{BX}$ - standardized value of 'X feature' for the 'B object' in the validation set; $x_{BX}$ - original value of 'X feature' for the 'B object' in the validation set; $m_X$ - mean value of 'X feature' for all objects in the training set; $s_X$ - standard deviation of 'X feature' for all objects in the training set.

9. [Validation set] Mapping

In this step, the standardized validation sets should be mapped (transformed) into principal components retaining all parameters from the PCA algorithm performed on the training set.

10. [Validation set] PCs cut-off

The selected number PCs for the validation set should be the same as in step 5 for each matrix: PC1 and PC2 for the nanoform characteristics matrix and PC1-PC3 for the cell lines matrix. Obtained scores values for samples in validation set are reported in the Table B1_4.

11. [Validation set] Predictions

In this step the developed model is used to predict biological response for samples in the validation set.

12. [Validation set] External validation

Predicted values from step 11 for each sample should be compared with the experimental responses to calculate validation statistical parameters.

The expected statistic values for the validation set should be as follow:

Accuracy: 0.833
Precision avg. macro: 0.889
Precision avg. micro: 0.833
Precision avg. weighted: 0.889
Recall avg. macro: 0.833
Recall avg. micro: 0.833
Recall avg. weighted: 0.833
F1_score avg. macro: 0.822
F1_score avg. micro: 0.833
F1_score avg. weighted: 0.822
MCC: 0.783

13. [New predictions] Defining nanoform and cell line

This step requires to define input parameters for the new prediction (nanoform characteristics and cell line characteristics). In order to ensure high reliability of predictions, the provided values of input parameters should be within the ranges of defined applicability domain defined below. The order of input parameters should be the same as in the training set.

Cell lines and nanoforms should be characterized by belonging to each of the specific groups listed below. Characteristics are expressed in a binary scale, i.e.: 1 - the cell line/nanoform belongs to the group, 0 - the cell line/nanoform does not belong to the group.

- cell line origin:

  - human [1 or 0]
  - mouse [1 or 0]
  - rat [1 or 0]

- cell type and organ of origin:

  - epithelium lung [1 or 0]
  - epithelium cervix [1 or 0]
  - macrophage monocyte [1 or 0]
  - epithelium liver [1 or 0]
  - neuron brain [1 or 0]
  - microglia brain [1 or 0]
  - macrophage lung [1 or 0]
  - macrophage bone marrow [1 or 0]
  - endothelium umbilical vein [1 or 0]
  - dendritic monocytes [1 or 0]

- germ layers the cells originate from:

  - endoderm [1 or 0]
  - ectoderm [1 or 0]
  - mesoderm [1 or 0]

- biological nature of cell used:

  - normal [1 or 0]
  - cancer [1 or 0]

Nanoforms should be characterized in terms of the following features and belong within the indicated value ranges:

- shape:

  ◦ spherical [1 or 0]
  ◦ irregular [1 or 0]
  ◦ inconclusive [1 or 0]

- size:

  ◦ min - minimum particle size, quantitative factor, derived from microscopic TEM images: 4.0-53.0 [nm]
  ◦ mean - mean particle size, quantitative factor, derived from microscopic TEM images: 5.0-110.0 [nm]
  ◦ max - maximum particle size, quantitative factor, derived from microscopic TEM images: 5.0-167.0 [nm]

- crystal structure:

  ◦ anatase - quantitative factor defining fraction of anatase form in the material: 0.0-1.0
  ◦ rutile - quantitative factor defining fraction of rutile form in the material: 0.0-1.0

- surface chemistry - qualitative factor defining presence of a coating on nanomaterial surface, expressed in a binary scale i.e.: 1 - presence of coating, 0 - absence of coating.

14. [New predictions] Scaling

The input parameters should be standardized by scaling the original values to the mean and standard deviation values calculated for the training set, according to the equation (Eq.3):

$$z_{CX} = \frac{x_{CX} - m_X}{s_X} \qquad (Eq.3)$$

Where: $z_{CX}$ - standardized value of 'X feature' for the input 'C sample'; $x_{BX}$ - original value of 'X feature' for the input 'C sample'; $m_X$ - mean value of 'X feature' for all objects in the training set; $s_X$ - standard deviation of 'X feature' for all objects in the training set.

15. [New predictions] Mapping

In this step, the standardized input samples should be mapped (transformed) into principal components retaining all parameters from the PCA algorithm performed on the training set.

16. [New predictions] PCs cut-off

The selected number PCs for the input samples should be the same as in step 5 for each matrix: PC1 and PC2 for the nanoform characteristics matrix and PC1-PC3 for the cell lines matrix.

17. [New predictions] Predictions

In this step the developed model is used to predict biological response for the input samples - new, untested nanoform(s), and defined cell line(s). As a result, the NOEC class is assigned to the defined samples.

Method B-2 - prediction of TNF-α NOEC class

Method B-2 employs a battery of common data curation and machine learning algorithms: standardization, dimensionality reduction based on principal component analysis, model development based on random forest classifier estimator. Said algorithms can be executed by means of any programming language and/or data analysis software.
The general scheme to recreate method B-2 of the invention is presented in Figure 2. The procedure is divided into 17 steps described below:

## 1. Datasets preparation

Method B-2 is based on an *in vitro* experimental data available for limited number of samples described with: i) matrix of cell lines characterization (Table B2_1), ii) matrix of nanoforms characterization (Table B2_2), and iii) the highest observed TiO$_2$ nanoform concentration which does not induce TNF-$\alpha$ in exposed mammalian cells, expressed as NOEC values ('no observed effect concentration') (Table B2_3). The observed NOEC is classified into three classes of concentration ranges:

[CLASS I] ≤ 10 $\mu$g/mL (5 samples)
10 $\mu$g/mL < [CLASS II] ≤ 100 $\mu$g/mL (13 samples)
100 $\mu$g/mL < [CLASS III] (5 samples)

## 2. Splitting of the datasets

In order to achieve the balanced split of NOEC classes within the training and validation sets, the available samples have been split according to the random split algorithm with 'stratify' parameter, which allows for the creation of representative sets. The recreation of the B-2 model requires the same split, and it is available in Table B2_1 - B2_4. The training set is used for calibration and verification of the model robustness (internal validation). The validation set is used for verification of the model prediction ability (external validation). The ratio between number of samples in the training and validation set is 8:3 (16 training set: 7 validation set).

The number of classes in training set is as follows:

[CLASS I] 3 samples
[CLASS II] 9 samples
[CLASS III] 4 samples

The number of classes in validation set is as follows:

[CLASS I] 2 sample
[CLASS II] 4 sample
[CLASS III] 1 samples

## 3. [Training set] Standardization

In this step, the matrices of cell lines and nanoforms characteristics should be standardized separately according to the equation (Eq.1):

$$z_{AX} = \frac{x_{AX} - m_X}{s_X} \qquad \text{(Eq.1)}$$

Where: $z_{AX}$ - standardized value of 'X feature' for the 'A object'; $x_{AX}$ - original value of 'X feature' for the 'A object'; $m_X$ - mean value of 'X feature' for all objects in the training set; $s_X$ - standard deviation of 'X feature' for all objects in the training set.

## 4. [Training set] Principal Component Analysis

The standardized matrices should be used in principal component analysis algorithm in order to compute principal components (PCs) for further model development.

## 5. [Training set] PCs reduction

Due to the requirement of balanced proportion between the samples in training set and variables used in the model (abovementioned 5:1 rule), the number of PCs should be reduced to the minimum number of PCs expressing at least 50% of summarized variance. In this case, for the nanoforms characteristics and cell lines matrices the optimal number of PCs are two (PC1 35.8%, PC2 30.0 %) and two (PC1 43.1%, PC2 33.9 %) respectively.

6. [Training set] Model calibration

Reduced number of PCs related to cell lines and nanoforms characteristics, and observed NOEC class are used for QSAR model development using machine learning method: random forest classifier. Obtained scores values for samples in training set are reported in the Table B2_4. The hyperparameters for reproducing the model should be as follows:
*n_estimators: 10; criterion: gini; max_depth: 5; min_samples_split: 2, min_samples_leaf: 1; min_weight_fraction_leaf. 0.0; max_features: sqrt; max_leaf_nodes: None; min_impurity_decrease: 0.0; bootstrap: True, oob_score: False, n_jobs: None, random_state: 42, verbose: 0, warm_start: False, class_weight: balanced, ccp_alpha: 0.0, max_samples: None*
At this step, a set of statistical parameters for training set should be as below:

Accuracy: 1.0
Precision avg. macro: 1.0
Precision avg. micro: 1.0
Precision avg. weighted: 1.0
Recall avg. macro: 1.0
Recall avg. micro: 1.0
Recall avg. weighted: 1.0
F1_score avg. macro: 1.0
F1_score avg. micro: 1.0
F1_score avg. weighted: 1.0
MCC: 1.0

7. [Training set] Internal validation

The internal validation of the developed model should be verified by performing stratified k-fold cross-validation. The hyperparameters should be as follow:
*cv: 5*
At this step, the statistical parameters for training set cross-validation should be as follow:
Accuracy: 0.533

8. [Validation set] Scaling

The validation set should be standardized by scaling the original values to the mean and standard deviation values calculated for the training set, according to the equation (Eq.2):

$$z_{BX} = \frac{x_{BX} - m_X}{s_X} \qquad (Eq.2)$$

Where: $z_{BX}$ - standardized value of 'X feature' for the 'B object' in the validation set; $x_{BX}$ - original value of 'X feature' for the 'B object' in the validation set; $m_X$ - mean value of 'X feature' for all objects in the training set; $s_X$ - standard deviation of 'X feature' for all objects in the training set.

9. [Validation set] Mapping

In this step, the standardized validation sets should be mapped (transformed) into principal components retaining all parameters from the PCA algorithm performed on the training set.

10. [Validation set] PCs cut-off

The selected number PCs for the validation set should be the same as in step 5 for each matrix: PC1 and PC2 for the nanoform characteristics matrix; and PC1 and PC2 for the cell lines matrix. Obtained scores values for samples in validation set are reported in the Table B2_4.

11. [Validation set] Predictions

In this step the developed model is used to predict biological response for samples in the validation set.

12. [Validation set] External validation

Predicted values from step 11 for each sample should be compared with the experimental responses to calculate validation statistical parameters.

The expected statistic values for the validation set should be as follow:

Accuracy: 0.857
Precision avg. macro: 0.933
Precision avg. micro: 0.857
Precision avg. weighted: 0.886
Recall avg. macro: 0.833
Recall avg. micro: 0.857
Recall avg. weighted: 0.857
F1_score avg. macro: 0.852
F1_score avg. micro: 0.857
F1_score avg. weighted: 0.841
MCC: 0.766

13. [New predictions] Defining nanoform and cell line

This step requires to define input parameters for the new prediction (nanoform characteristics and cell line characteristics). In order to ensure high reliability of predictions, the provided values of input parameters should be within the ranges of defined applicability domain defined below. The order of input parameters should be the same as in the training set.

Cell lines and nanoforms should be characterized by belonging to each of the specific groups listed below. Characteristics are expressed in a binary scale, i.e.: 1 - the cell line/nanoform belongs to the group, 0 - the cell line/nanoform does not belong to the group.

- cell line origin:

  - human [1 or 0]
  - mouse [1 or 0]
  - rat [1 or 0]

- cell type and organ of origin:

  - macrophage monocyte [1 or 0]
  - epithelium lung [1 or 0]
  - macrophage lung alveolar [1 or 0]
  - macrophage bone marrow [1 or 0]

- germ layers the cells originate from:

  - endoderm [1 or 0]
  - mesoderm [1 or 0]

- biological nature of cell used:

  - normal [1 or 0]
  - cancer [1 or 0]

Nanoforms should be characterized in terms of the following features and belong within the indicated value ranges:

- shape:

  - spherical [1 or 0]
  - elongated [1 or 0]
  - inconclusive [1 or 0]

- size:

  - min - minimum particle size, quantitative factor, derived from microscopic TEM images: 5.3-100.0 [nm]
  - mean - mean particle size, quantitative factor, derived from microscopic TEM images: 6.0-110.0 [nm]
  - max - maximum particle size, quantitative factor, derived from microscopic TEM images: 6.7-167.0 [nm]

- crystal structure:

  - anatase - quantitative factor defining fraction of anatase form in the material: 0.0-1.0
  - rutile - quantitative factor defining fraction of rutile form in the material: 0.0-1.0

- surface chemistry - qualitative factor defining presence of a coating on nanomaterial surface, expressed in a binary scale i.e.: 1 - presence of coating, 0 - absence of coating.

14. [New predictions] Scaling

The input parameters should be standardized by scaling the original values to the mean and standard deviation values calculated for the training set, according to the equation (Eq.3):

$$z_{CX} = \frac{x_{CX} - m_X}{s_X} \qquad (Eq.3)$$

Where: $z_{CX}$ - standardized value of 'X feature' for the input 'C sample'; $x_{BX}$ - original value of 'X feature' for the input 'C sample'; $m_X$ - mean value of 'X feature' for all objects in the training set; $s_X$ - standard deviation of 'X feature' for all objects in the training set.

15. [New predictions] Mapping

In this step, the standardized input samples should be mapped (transformed) into principal components retaining all parameters from the PCA algorithm performed on the training set.

16. [New predictions] PCs cut-off

The selected number PCs for the input samples should be the same as in step 5 for each matrix: PC1 and PC2 for the nanoform characteristics matrix, and PC1 and PC2 for the cell lines matrix.

17. [New predictions] Predictions

In this step the developed model is used to predict biological response for the input samples - new, untested nanoform(s), and defined cell line(s). As a result, the NOEC class is assigned to the defined samples.

The invention is described in much details in the examples and drawings where on Fig 1 it is shown scheme of approach A (method A-1, A-2), on figure 2 - scheme of approach B (method B-1, B-2).

EXAMPLES

The developed QSAR models - methods A-1, A-2, B-1 and B-2 - are used for prediction of expression of strong proinflammatory cytokines: interleukin-6 (IL-6), and Tumor Necrosis Factor-$\alpha$ (TNF-$\alpha$) in defined mammalian cell lines treated with new, untested $TiO_2$ nanoforms (new samples). These methods are based on the identified relationships between cell line and nanoforms characteristics (and concentration in case of methods A-1 and A-2) and biological responses observed for samples used during the calibration of the methods (samples used in the training sets for a proper method).

The inventions' use cases are presented below. These examples present diverse samples (nanoforms and cell line characteristics) for which the QSAR models were applied to predict the induction of cytokines (methods A-1 and A-2) or NOEC class (methods B-1 and B-2).

Example 1 - prediction case study of method A-1

The presented case study is performed on new, untested $TiO_2$ nanoforms, defined cell line and concentration for which the proinflammatory induction of IL-6 is unknown. The prediction of unknown activity requires the execution of all 17 steps included in method A-1 and presented on the Figure 1.

After completing the steps 1-12 of a method A-1 and correct reconstruction of the QSAR model, confirmed with the converged statistical parameters; new nanoform, cell line (BEAS-2B) and concentration was defined (step 13):

Cell line characteristics:

- cell line origin:

  ◦ human: 1
  ◦ mouse: 0
  ◦ rat: 0

- cell type and organ of origin:

  ◦ epithelium lung: 1
  ◦ epithelium cervix: 0
  ◦ macrophage monocyte: 0
  ◦ epithelium liver: 0
  ◦ neuron brain: 0
  ◦ microglial brain: 0
  ◦ macrophage lung: 0
  ◦ macrophage bone marrow: 0
  ◦ endothelium umbilical vein: 0
  ◦ dendritic monocytes: 0

- germ layers the cells originate from:

  ◦ endoderm:1
  ◦ ectoderm: 0
  ◦ mesoderm:0

- biological nature of cell used:

  ◦ normal: 1
  ◦ cancer:0

Nanoform characteristics:

- shape:

  ◦ spherical: 0
  ◦ irregular: 1
  ◦ inconclusive: 0

- size:

  ◦ Min - 50.0 [nm]
  ◦ Mean - 65.0 [nm]
  ◦ Max-70.0 [nm]

- crystal structure:

  ◦ anatase: 0.8
  ◦ rutile: 0.2

- surface chemistry: 0

Concentrations: 50.00 [$\mu$g/mL]

Subsequently, the execution of scaling, mapping and PCs cut-off (steps 14-16) for newly defined sample (Example_1), resulted in obtaining inputs, which together with the defined 'Concentration' were used for predictions with the A-1 model (Table 1):

*Table 1. Inputs of newly defined nanoform, cell line and concentration for the predictions with A-1 model.*

| Sample ID | Nanoform characteristic | | Cell line characteristic | | | Concentration [$\mu$g/mL] |
|---|---|---|---|---|---|---|
| | PC1 | PC2 | PC1 | PC2 | PC3 | |
| Example_1 | 2.644948 | -0.373139 | 2.428216 | -0.922156 | 0.492276 | 50.00 |

The obtained results i.e., score values of PC1 and PC2 for nanoform characteristics, PC1, PC2 and PC3 for cell line characteristics, and defined concentration, were then used as inputs for the developed decision tree classifier. The application of a recreated A-1 model (step 17) resulted in: <u>negative response</u> of proinflammatory expression of IL-6 for the newly defined sample (Example_1).

Example 2 - prediction case study of method A-2

The presented case study is performed on new, untested TiO$_2$ nanoforms, defined cell line and concentration for which the proinflammatory induction of TNF-$\alpha$ is unknown. The prediction of unknown activity requires the execution of all 17 steps included in method A-2 and presented on the Figure 1.
After completing the steps 1-12 of a method A-2 and correct reconstruction of the QSAR model, confirmed with the converged statistical parameters; new nanoform, cell line (BEAS-2B) and concentration were defined (step 13):

Cell line characteristics:

- cell line origin:

  ◦ human: 1
  ◦ mouse: 0
  ◦ rat: 0

- cell type and organ of origin:

  ◦ macrophage monocyte: 0
  ◦ epithelium lung: 1
  ◦ macrophage lung alveolar: 0
  ◦ macrophage bone marrow: 0

- germ layers the cells originate from:

  ◦ endoderm:1
  ◦ mesoderm: 0

- biological nature of cell used:

  ◦ normal: 1
  ◦ cancer:0

Nanoforms characteristics:

- shape:

  ◦ spherical: 1
  ◦ elongated: 0
  ◦ inconclusive: 0

- size:

  ◦ Min: 45.0 [nm]
  ◦ Mean: 50.0 [nm]
  ◦ Max: 55.0 [nm]

- crystal structure:

  ◦ anatase: 1.0
  ◦ rutile: 0.0

- Surface chemistry: 0

Concentration: 40.00 [$\mu$g/mL].

Subsequently, the execution of scaling, mapping and PCs cut-off (steps 14-16) for newly defined sample (Example_2), resulted in obtaining inputs, which together with the defined 'Concentration' were used for prediction with the A-2 model (Table 2):

*Table 2. Inputs of newly defined nanoform, cell line and concentration for the prediction with A-2 model.*

| Sample ID | Nanoform characteristic | | Cell line characteristic | | Concentration [$\mu$g/mL] |
|---|---|---|---|---|---|
| | PC1 | PC2 | PC1 | PC2 | |
| Example_2 | -0.99313 | 1.186227 | 1.055688 | -1.343931 | 40.00 |

The obtained results i.e., score values of PC1 and PC2 for nanoform characteristics, PC1 and PC2 for cell line characteristics, and defined concentration, were then used as inputs for the developed k-Nearest Neighbors classifier. The application of a recreated A-2 model (step 17) resulted in: negative response of proinflammatory expression of TNF-$\alpha$ for the newly defined sample (Example_2).

Example 3 - prediction case study of method B-1

The presented case study is performed on new, untested TiO$_2$ nanoforms and defined cell line for which the highest TiO$_2$ nanoform concentration which does not induce IL-6 in exposed mammalian cells ('no observed effect concentration', NOEC value), is unknown. The prediction of unknown activity requires the execution of all 17 steps included in method B-1 and presented on the Figure 2.

After completing the steps 1-12 of a method B-1 and correct reconstruction of the QSAR model, confirmed with the converged statistical parameters; new nanoform and cell line (A549) were defined (step 13):

Cell line characteristics:

- cell line origin:

  ◦ human: 1
  ◦ mouse: 0
  ◦ rat: 0

- cell type and organ of origin:

○ epithelium lung: 1
○ epithelium cervix: 0
○ macrophage monocyte: 0
○ epithelium liver: 0
○ neuron brain: 0
○ microglial brain: 0
○ macrophage lung: 0
○ macrophage bone marrow: 0
○ endothelium umbilical vein: 0
○ dendritic monocytes: 0

■ germ layers the cells originate from:

○ endoderm:1
○ ectoderm: 0
○ mesoderm:0

■ biological nature of cell used:

○ normal: 0
○ cancer:1

Nanoform characteristics:

■ shape:

○ spherical: 1
○ irregular: 0
○ inconclusive: 0

■ size:

○ Min: 13.0 [nm]
○ Mean: 15.0 [nm]
○ Max: 17.0 [nm]

■ crystal structure:

○ anatase: 1.0
○ rutile: 0.0

■ Surface chemistry: 0

Subsequently, the execution of scaling, mapping and PCs cut-off (steps 14-16) for newly defined sample (Example_3), resulted in obtaining inputs for the B-1 model (Table 3):

*Table 3. Inputs of newly defined nanoform and cell line for the prediction with B-1 model.*

| Sample ID | Nanoform characteristic | | Cell line characteristic | | |
|---|---|---|---|---|---|
| | PC1 | PC2 | PC1 | PC2 | PC3 |
| Example_3 | -0.8841 | 1.376875 | 2.553747 | 1.120093 | -0.337689 |

The obtained results i.e., score values of PC1 and PC2 for nanoform characteristics, and PC1, PC2 and PC3 for cell line characteristics, were then used as inputs for the developed decision tree classifier. The application of a recreated B-1 model (step 17) resulted in a predicted NOEC value of IL-6: [CLASS I] ≤ 10 μg/mL for the newly defined sample (Example_3).

Example 4 - prediction case study of method B-2

The presented case study is performed on a new, untested $TiO_2$ nanoforms and defined cell line for which the highest $TiO_2$ nanoform concentration which does not induce TNF-$\alpha$ in exposed mammalian cells ('no observed effect concentration', NOEC value), is unknown. The prediction of unknown activity requires the execution of all 17 steps included in method B-2 and presented on the Figure 2.

After completing the steps 1-12 of a method B-2 and correct reconstruction of the QSAR model, confirmed with the converged statistical parameters; new nanoform and cell line (RAW264.7) were defined (step 13):

Cell line characteristics:

- cell line origin:

  ○ human: 0
  ○ mouse: 1
  ○ rat: 0

- cell type and organ of origin:

  ○ macrophage monocyte: 1
  ○ epithelium lung: 0
  ○ macrophage lung alveolar: 0
  ○ macrophage bone marrow: 0

- germ layers the cells originate from:

  ○ endoderm:0
  ○ mesoderm: 1

- biological nature of cell used:

  ○ normal: 0
  ○ cancer:1

Nanoforms characteristics:

- shape:

  ○ spherical: 1
  ○ elongated: 0
  ○ inconclusive: 0

- size:

  ○ Min: 28.0 [nm]
  ○ Mean: 31.0 [nm]
  ○ Max: 34.0 [nm]

- crystal structure:

  ○ anatase: 0.0
  ○ rutile: 1.0

- Surface chemistry: 0

Subsequently, the execution of scaling, mapping and PCs cut-off (steps 14-16) for newly defined sample (Example_4), resulted in obtaining inputs for the B-2 model (Table 4):

*Table 4. Inputs of newly defined nanoform, and cell line for the prediction with B-2 model.*

| Sample ID | Nanoform characteristic | | Cell line characteristic | |
|---|---|---|---|---|
| | PC1 | PC2 | PC1 | PC2 |
| Example_4 | 1.117794 | 0.248237 | -0.43664 | 0.535558 |

The obtained results i.e., score values of PC1 and PC2 for nanoform characteristics, PC1 and PC2 for cell line characteristics, were then used as inputs for the developed random forest classifier. The application of a recreated B-2 model (step 17) resulted in a predicted NOEC value of TNF-$\alpha$: [CLASS III] > 100 $\mu$g/mL for the newly defined sample (Example_4).

Table A1_1. Cell line characteristics of samples used in development and validation of method A-1 (T – training set, V – validation set).

| ID | Cell line | Test assay | Split | Human | Mouse | Rat | Epithelium lung | Macrophage lung | Macrophage monocyte | Dendritic monocytes | Epithelium cervix | Epithelium liver | Neuron brain | Microglia brain | Macrophage bone marrow | Endothelium umbilical vein | Mesoderm | Endoderm | Ectoderm | Normal | Cancer |
|---|---|---|---|---|---|---|---|---|---|---|---|---|---|---|---|---|---|---|---|---|---|
| 0 | HUVEC | ELISA | T | 1 | 0 | 0 | 0 | 0 | 0 | 0 | 0 | 0 | 0 | 0 | 0 | 1 | 1 | 0 | 0 | 1 | 0 |
| 1 | NCI-H292 | RT-PCR | T | 1 | 0 | 0 | 1 | 0 | 0 | 0 | 0 | 0 | 0 | 0 | 0 | 0 | 0 | 1 | 0 | 0 | 1 |
| 2 | HeLa | RT-PCR | T | 1 | 0 | 0 | 0 | 0 | 0 | 0 | 1 | 0 | 0 | 0 | 0 | 0 | 1 | 0 | 0 | 0 | 1 |
| 3 | THP-1 | RT-PCR | T | 1 | 0 | 0 | 0 | 0 | 1 | 0 | 0 | 0 | 0 | 0 | 0 | 0 | 1 | 0 | 0 | 0 | 1 |
| 4 | HepG2 | RT-PCR | T | 1 | 0 | 0 | 0 | 0 | 0 | 0 | 0 | 1 | 0 | 0 | 0 | 0 | 0 | 1 | 0 | 0 | 1 |
| 5 | A549 | ELISA | T | 1 | 0 | 0 | 1 | 0 | 0 | 0 | 0 | 0 | 0 | 0 | 0 | 0 | 0 | 1 | 0 | 0 | 1 |
| 6 | BEAS-2B | ELISA | T | 1 | 0 | 0 | 1 | 0 | 0 | 0 | 0 | 0 | 0 | 0 | 0 | 0 | 0 | 1 | 0 | 1 | 0 |
| 7 | TT1 | ELISA | T | 1 | 0 | 0 | 1 | 0 | 0 | 0 | 0 | 0 | 0 | 0 | 0 | 0 | 0 | 1 | 0 | 1 | 0 |
| 8 | TT1 | ELISA | T | 1 | 0 | 0 | 1 | 0 | 0 | 0 | 0 | 0 | 0 | 0 | 0 | 0 | 0 | 1 | 0 | 1 | 0 |
| 9 | A549 | ELISA | V | 1 | 0 | 0 | 1 | 0 | 0 | 0 | 0 | 0 | 0 | 0 | 0 | 0 | 0 | 1 | 0 | 0 | 1 |
| 10 | A549 | ELISA | T | 1 | 0 | 0 | 1 | 0 | 0 | 0 | 0 | 0 | 0 | 0 | 0 | 0 | 0 | 1 | 0 | 0 | 1 |
| 11 | BEAS-2B | ELISA | V | 1 | 0 | 0 | 1 | 0 | 0 | 0 | 0 | 0 | 0 | 0 | 0 | 0 | 0 | 1 | 0 | 1 | 0 |
| 12 | BEAS-2B | ELISA | T | 1 | 0 | 0 | 1 | 0 | 0 | 0 | 0 | 0 | 0 | 0 | 0 | 0 | 0 | 1 | 0 | 1 | 0 |
| 13 | A549 | RT-PCR | T | 1 | 0 | 0 | 1 | 0 | 0 | 0 | 0 | 0 | 0 | 0 | 0 | 0 | 0 | 1 | 0 | 0 | 1 |
| 14 | RAW 264.7 | RT-PCR | T | 0 | 1 | 0 | 0 | 0 | 1 | 0 | 0 | 0 | 0 | 0 | 0 | 0 | 1 | 0 | 0 | 0 | 1 |
| 15 | N2a | ELISA | T | 0 | 1 | 0 | 0 | 0 | 0 | 0 | 0 | 0 | 1 | 0 | 0 | 0 | 0 | 0 | 1 | 0 | 1 |
| 16 | BV-2 | ELISA | T | 0 | 1 | 0 | 0 | 0 | 0 | 0 | 0 | 0 | 0 | 1 | 0 | 0 | 1 | 0 | 0 | 0 | 1 |

| No. | Cell | Method | T/V | | | | | | | | | | | | | | | | | | |
|---|---|---|---|---|---|---|---|---|---|---|---|---|---|---|---|---|---|---|---|---|---|
| 17 | MH-S | Milliplex Map Kit | T | 0 | 1 | 0 | 0 | 1 | 0 | 0 | 0 | 0 | 0 | 0 | 0 | 0 | 1 | 0 | 0 | 1 | 0 |
| 18 | BEAS-2B | ELISA | T | 1 | 0 | 0 | 1 | 0 | 0 | 0 | 0 | 0 | 0 | 0 | 0 | 0 | 0 | 1 | 0 | 1 | 0 |
| 19 | BEAS-2B | ELISA | T | 1 | 0 | 0 | 1 | 0 | 0 | 0 | 0 | 0 | 0 | 0 | 0 | 0 | 0 | 1 | 0 | 1 | 0 |
| 20 | RAW 264.7 | ELISA | T | 0 | 1 | 0 | 0 | 0 | 1 | 0 | 0 | 0 | 0 | 0 | 0 | 0 | 1 | 0 | 0 | 0 | 1 |
| 21 | RAW 264.7 | ELISA | T | 0 | 1 | 0 | 0 | 0 | 1 | 0 | 0 | 0 | 0 | 0 | 0 | 0 | 1 | 0 | 0 | 0 | 1 |
| 22 | BMDMs | RT-PCR | T | 0 | 1 | 0 | 0 | 0 | 0 | 0 | 0 | 0 | 0 | 0 | 0 | 1 | 1 | 0 | 0 | 1 | 0 |
| 23 | BMDMs | RT-PCR | T | 0 | 1 | 0 | 0 | 0 | 0 | 0 | 0 | 0 | 0 | 0 | 0 | 1 | 1 | 0 | 0 | 1 | 0 |
| 24 | BMDMs | RT-PCR | T | 0 | 1 | 0 | 0 | 0 | 0 | 0 | 0 | 0 | 0 | 0 | 0 | 1 | 1 | 0 | 0 | 1 | 0 |
| 25 | BMDMs | RT-PCR | V | 0 | 1 | 0 | 0 | 0 | 0 | 0 | 0 | 0 | 0 | 0 | 0 | 1 | 1 | 0 | 0 | 1 | 0 |
| 26 | BMDMs | RT-PCR | V | 0 | 1 | 0 | 0 | 0 | 0 | 0 | 0 | 0 | 0 | 0 | 0 | 1 | 1 | 0 | 0 | 1 | 0 |
| 27 | BMDMs | RT-PCR | V | 0 | 1 | 0 | 0 | 0 | 0 | 0 | 0 | 0 | 0 | 0 | 0 | 1 | 1 | 0 | 0 | 1 | 0 |
| 28 | 16HBE14o- | ELISA | V | 1 | 0 | 0 | 1 | 0 | 0 | 0 | 0 | 0 | 0 | 0 | 0 | 0 | 0 | 1 | 0 | 1 | 0 |
| 29 | DCs | Bio-Plex system | T | 1 | 0 | 0 | 0 | 0 | 0 | 1 | 0 | 0 | 0 | 0 | 0 | 0 | 1 | 0 | 0 | 1 | 0 |
| 30 | alveolar macrophages | radio-immuno assay | T | 0 | 0 | 1 | 0 | 1 | 0 | 0 | 0 | 0 | 0 | 0 | 0 | 0 | 1 | 0 | 0 | 1 | 0 |
| 31 | alveolar macrophages | radio-immuno assay | V | 0 | 0 | 1 | 0 | 1 | 0 | 0 | 0 | 0 | 0 | 0 | 0 | 0 | 1 | 0 | 0 | 1 | 0 |
| 32 | alveolar macrophages | radio-immuno assay | T | 0 | 0 | 1 | 0 | 1 | 0 | 0 | 0 | 0 | 0 | 0 | 0 | 0 | 1 | 0 | 0 | 1 | 0 |

BMDMs- Bone marrow derived macrophages
TT1 cells - alveolar type- I-like epithelial cells

EP 4 376 011 A1

N2a - (Neuro2a) neuroblastoma cells
DCs - human monocyte–derived Dendritic Cells
HUVEC with PBMCs- DCS equivalent - Peripheral Tissue Equivalent Module

Table A1_2. Nanoform characteristics of samples used in development and validation of method A-1 (T - training set, V- validation set).

| ID | Split | Spherical | Irregular | Inconclusive | Minimum particle size [nm] | Mean particle size [nm] | Maximum particle size [nm] | Crystal structure - anatase | Crystal structure - rutile | Surface chemistry |
|---|---|---|---|---|---|---|---|---|---|---|
| 0 | T | 0 | 0 | 1 | 4.0 | 7.0 | 8.0 | 0.00 | 1.00 | 0 |
| 1 | T | 0 | 0 | 1 | 49.6 | 49.6 | 49.6 | 0.80 | 0.20 | 1 |
| 2 | T | 0 | 0 | 1 | 49.6 | 49.6 | 49.6 | 0.80 | 0.20 | 1 |
| 3 | T | 0 | 0 | 1 | 49.6 | 49.6 | 49.6 | 0.80 | 0.20 | 1 |
| 4 | T | 0 | 0 | 1 | 49.6 | 49.6 | 49.6 | 0.80 | 0.20 | 1 |
| 5 | T | 1 | 0 | 0 | 26.8 | 43.8 | 60.8 | 0.79 | 0.21 | 0 |
| 6 | T | 1 | 0 | 0 | 26.8 | 43.8 | 60.8 | 0.79 | 0.21 | 0 |
| 7 | T | 1 | 0 | 0 | 6.0 | 8.0 | 10.0 | 1.00 | 0.00 | 0 |
| 8 | T | 1 | 0 | 0 | 5.0 | 8.5 | 12.0 | 0.00 | 1.00 | 0 |
| 9 | V | 1 | 0 | 0 | 26.8 | 43.8 | 60.8 | 0.79 | 0.21 | 0 |
| 10 | T | 0 | 1 | 0 | 23.9 | 76.6 | 129.3 | 0.19 | 0.81 | 0 |
| 11 | V | 1 | 0 | 0 | 26.8 | 43.8 | 60.8 | 0.79 | 0.21 | 0 |
| 12 | T | 0 | 1 | 0 | 23.9 | 76.6 | 129.3 | 0.19 | 0.81 | 0 |
| 13 | T | 1 | 0 | 0 | 23.0 | 23.0 | 23.0 | 0.50 | 0.50 | 0 |
| 14 | T | 1 | 0 | 0 | 23.0 | 23.0 | 23.0 | 0.50 | 0.50 | 0 |
| is | T | 0 | 0 | 1 | 5.8 | 5.8 | 5.8 | 1.00 | 0.00 | 0 |
| 16 | T | 0 | 0 | 1 | 5.8 | 5.8 | 5.8 | 1.00 | 0.00 | 0 |
| 17 | T | 0 | 0 | 1 | 10.0 | 10.0 | 10.0 | 1.00 | 0.00 | 0 |
| 18 | T | 0 | 0 | 1 | 53.0 | 110.0 | 167.0 | 1.00 | 0.00 | 0 |
| 19 | T | 0 | 0 | 1 | 5.3 | 6.0 | 6.7 | 1.00 | 0.00 | 0 |
| 20 | T | 0 | 0 | 1 | 53.0 | 110.0 | 167.0 | 1.00 | 0.00 | 0 |
| 21 | T | 0 | 0 | 1 | 5.3 | 6.0 | 6.7 | 1.00 | 0.00 | 0 |
| 22 | T | 0 | 0 | 1 | 10.0 | 10.0 | 10.0 | 1.00 | 0.00 | 0 |
| 23 | T | 0 | 0 | 1 | 50.0 | 50.0 | 50.0 | 1.00 | 0.00 | 0 |

35

| ID | Split | Spherical | Irregular | Inconclusive | Minimum particle size [nm] | Mean particle size [nm] | Maximum particle size [nm] | Crystal structure - anatase | Crystal structure - rutile | Surface chemistry |
|---|---|---|---|---|---|---|---|---|---|---|
| 24 | T | 0 | 0 | 1 | 12.0 | 15.0 | 18.0 | 0.80 | 0.20 | 0 |
| 25 | V | 0 | 0 | 1 | 10.0 | 10.0 | 10.0 | 1.00 | 0.00 | 0 |
| 26 | V | 0 | 0 | 1 | 12.0 | 13.5 | 15.0 | 1.00 | 0.00 | 0 |
| 27 | V | 0 | 0 | 1 | 16.0 | 16.5 | 17.0 | 1.00 | 0.00 | 0 |
| 28 | V | 0 | 0 | 1 | 15.0 | 15.0 | 15.0 | 1.00 | 0.00 | 0 |
| 29 | T | 0 | 0 | 1 | 7.0 | 8.5 | 10.0 | 1.00 | 0.00 | 0 |
| 30 | T | 0 | 0 | 1 | 5.0 | 5.0 | 5.0 | 1.00 | 0.00 | 0 |
| 31 | V | 0 | 0 | 1 | 21.0 | 21.0 | 21.0 | 0.80 | 0.20 | 0 |
| 32 | T | 0 | 0 | 1 | 50.0 | 50.0 | 50.0 | 0.00 | 1.00 | 0 |

*Table A1_3. Concentrations of samples used in development and validation of method A-1 (T - training set, V - validation set).*

| ID | Split | Concentration [μg/mL] |
|---|---|---|
| 0 | T | 16.00 |
| 1 | T | 75.00 |
| 2 | T | 75.00 |
| 3 | T | 75.00 |
| 4 | T | 75.00 |
| 5 | T | 5.00 |
| 6 | T | 40.00 |
| 7 | T | 10.00 |
| 8 | T | 1.00 |
| 9 | V | 5.00 |
| 10 | T | 10.00 |
| 11 | V | 40.00 |
| 12 | T | 40.00 |
| 13 | T | 200.00 |
| 14 | T | 200.00 |
| is | T | 100.00 |
| 16 | T | 100.00 |
| 17 | T | 12.50 |
| 18 | T | 100.00 |
| 19 | T | 100.00 |
| 20 | T | 100.00 |
| 21 | T | 10.00 |
| 22 | T | 10.00 |
| 23 | T | 10.00 |
| 24 | T | 128.00 |
| 25 | V | 128.00 |
| 26 | V | 128.00 |
| 27 | V | 128.00 |
| 28 | V | 38.00 |
| 29 | T | 0.08 |
| 30 | T | 75.00 |
| 31 | V | 18.50 |
| 32 | T | 300.00 |

*Table A1_4. Proinflammatory induction of IL-6 of samples used in development and validation of method A-1 (1 - positive, 0 - negative response) (T - training set, V - validation set).*

| ID | Split | Proinflammation effect |
|---|---|---|
| 0 | T | 0 |
| 1 | T | 1 |
| 2 | T | 0 |
| 3 | T | 0 |
| 4 | T | 0 |
| 5 | T | 1 |
| 6 | T | 0 |
| 7 | T | 1 |
| 8 | T | 1 |
| 9 | V | 1 |
| 10 | T | 1 |
| 11 | V | 0 |
| 12 | T | 0 |
| 13 | T | 0 |
| 14 | T | 0 |
| is | T | 1 |
| 16 | T | 0 |
| 17 | T | 1 |
| 18 | T | 0 |
| 19 | T | 1 |
| 20 | T | 1 |
| 21 | T | 1 |
| 22 | T | 1 |
| 23 | T | 1 |
| 24 | T | 0 |
| 25 | V | 0 |
| 26 | V | 0 |
| 27 | V | 0 |
| 28 | V | 1 |
| 29 | T | 1 |
| 30 | T | 1 |
| 31 | V | 1 |
| 32 | T | 1 |

*Table A1_5. Scores values of samples used in development and validation of method A-1 (T - training set, V - validation set).*

| ID | Split | Nanoform characteristic | | Cell line characteristic | | |
|----|-------|------|------|------|------|------|
|    |       | PC1 | PC2 | PC1 | PC2 | PC3 |
| 0 | T | 0.203124 | 1.899862 | -0.20448 | -1.67476 | -0.60785 |
| 1 | T | 0.196617 | -2.16964 | 2.380848 | 1.098418 | -0.22164 |
| 2 | T | 0.196617 | -2.16964 | -0.26588 | 1.329516 | -2.03308 |
| 3 | T | 0.196617 | -2.16964 | -0.70477 | 1.569461 | -1.86911 |
| 4 | T | 0.196617 | -2.16964 | 1.97099 | 1.719838 | -0.76428 |
| 5 | T | 0.604231 | 1.064841 | 2.380848 | 1.098418 | -0.22164 |
| 6 | T | 0.604231 | 1.064841 | 2.428216 | -0.92216 | 0.492276 |
| 7 | T | -1.12399 | 1.867196 | 2.428216 | -0.92216 | 0.492276 |
| 8 | T | 1.05753 | 3.620918 | 2.428216 | -0.92216 | 0.492276 |
| 9 | V | 0.604231 | 1.064841 | 2.380848 | 1.098418 | -0.22164 |
| 10 | T | 4.278187 | 0.810653 | 2.380848 | 1.098418 | -0.22164 |
| 11 | V | 0.604231 | 1.064841 | 2.428216 | -0.92216 | 0.492276 |
| 12 | T | 4.278187 | 0.810653 | 2.428216 | -0.92216 | 0.492276 |
| 13 | T | 0.543932 | 2.111411 | 2.380848 | 1.098418 | -0.22164 |
| 14 | T | 0.543932 | 2.111411 | -2.33045 | 1.896636 | -1.37695 |
| is | T | -1.97785 | 0.136975 | -1.85573 | 4.215994 | 5.928165 |
| 16 | T | -1.97785 | 0.136975 | -2.37312 | 1.815973 | -1.05056 |
| 17 | T | -1.81815 | -0.02994 | -2.30882 | -1.76365 | 0.519983 |
| 18 | T | 1.645945 | -3.04167 | 2.428216 | -0.92216 | 0.492276 |
| 19 | T | -1.9744 | 0.140847 | 2.428216 | -0.92216 | 0.492276 |
| 20 | T | 1.645945 | -3.04167 | -2.33045 | 1.896636 | -1.37695 |
| 21 | T | -1.9744 | 0.140847 | -2.33045 | 1.896636 | -1.37695 |
| 22 | T | -1.81815 | -0.02994 | -2.37284 | -1.24083 | 0.410219 |
| 23 | T | -0.29722 | -1.61966 | -2.37284 | -1.24083 | 0.410219 |
| 24 | T | -1.81815 | -0.02994 | -2.37284 | -1.24083 | 0.410219 |
| 25 | V | -1.21155 | 0.171655 | -2.37284 | -1.24083 | 0.410219 |
| 26 | V | -1.69397 | -0.14371 | -2.37284 | -1.24083 | 0.410219 |
| 27 | V | -1.57397 | -0.27983 | -2.37284 | -1.24083 | 0.410219 |
| 28 | V | -1.62803 | -0.22866 | 2.428216 | -0.92216 | 0.492276 |
| 29 | T | -1.88408 | 0.055 | -0.20448 | -1.67476 | -0.60785 |
| 30 | T | -0.96561 | -0.11746 | -2.01827 | -3.18288 | 0.658838 |
| 31 | V | -2.00827 | 0.168769 | -2.01827 | -3.18288 | 0.658838 |
| 32 | T | 1.874188 | 0.128605 | -2.01827 | -3.18288 | 0.658838 |

Table A2_1. Cell line characteristics of samples used in development and validation of method A-2 (T – training set, V – validation set).

| ID | Cell line | Test assay | Split | Human | Mouse | Rat | Epithelium lung | Macrophage lung | Macrophage monoctes | Macrophage bone marrow | Mesoderm | Endoderm | Normal | Cancer |
|----|-----------|------------|-------|-------|-------|-----|-----------------|-----------------|---------------------|------------------------|----------|----------|--------|--------|
| 0 | RAW264.7 | ELISA | T | 0 | 1 | 0 | 0 | 0 | 1 | 0 | 1 | 0 | 0 | 1 |
| 1 | RAW264.7 | ELISA | T | 0 | 1 | 0 | 0 | 0 | 1 | 0 | 1 | 0 | 0 | 1 |
| 2 | RAW264.7 | ELISA | T | 0 | 1 | 0 | 0 | 0 | 1 | 0 | 1 | 0 | 0 | 1 |
| 3 | BEAS-2B | ELISA | T | 1 | 0 | 0 | 1 | 0 | 0 | 0 | 0 | 1 | 1 | 0 |
| 4 | BEAS-2B | ELISA | V | 1 | 0 | 0 | 1 | 0 | 0 | 0 | 0 | 1 | 1 | 0 |
| 5 | BEAS-2B | ELISA | T | 1 | 0 | 0 | 1 | 0 | 0 | 0 | 0 | 1 | 1 | 0 |
| 6 | BEAS-2B | ELISA | T | 1 | 0 | 0 | 1 | 0 | 0 | 0 | 0 | 1 | 1 | 0 |
| 7 | A549 | ELISA | T | 1 | 0 | 0 | 1 | 0 | 0 | 0 | 0 | 1 | 0 | 1 |
| 8 | BEAS-2B | ELISA | T | 1 | 0 | 0 | 1 | 0 | 0 | 0 | 0 | 1 | 1 | 0 |
| 9 | NR8383 | ELISA | T | 0 | 0 | 1 | 0 | 1 | 0 | 0 | 1 | 0 | 1 | 0 |
| 10 | RAW 264.7 | RT-PCR | T | 0 | 1 | 0 | 0 | 0 | 1 | 0 | 1 | 0 | 0 | 1 |
| 11 | BMDMs | RT-PCR | V | 0 | 1 | 0 | 0 | 0 | 0 | 1 | 1 | 0 | 1 | 0 |
| 12 | RAW 264.7 | ELISA | T | 0 | 1 | 0 | 0 | 0 | 1 | 0 | 1 | 0 | 0 | 1 |
| 13 | BEAS-2B | ELISA | T | 1 | 0 | 0 | 1 | 0 | 0 | 0 | 0 | 1 | 1 | 0 |
| 14 | BEAS-2B | ELISA | T | 1 | 0 | 0 | 1 | 0 | 0 | 0 | 0 | 1 | 1 | 0 |
| 15 | RAW 264.7 | ELISA | T | 0 | 1 | 0 | 0 | 0 | 1 | 0 | 1 | 0 | 0 | 1 |
| 16 | RAW 264.7 | ELISA | V | 0 | 1 | 0 | 0 | 0 | 1 | 0 | 1 | 0 | 0 | 1 |
| 17 | BMDMs | RT-PCR | T | 0 | 1 | 0 | 0 | 0 | 0 | 1 | 1 | 0 | 1 | 0 |

EP 4 376 011 A1

| # | Cell | Method | | | | | | | | | | | | |
|---|------|--------|---|---|---|---|---|---|---|---|---|---|---|---|
| 18 | BMDMs | RT-PCR | T | 0 | 1 | 0 | 1 | 1 | 0 | 0 | 0 | 0 | 1 | 0 |
| 19 | NR8383 | ELISA | V | 0 | 1 | 0 | 1 | 0 | 0 | 1 | 0 | 1 | 0 | 0 |
| 20 | NR8383 | ELISA | V | 0 | 1 | 0 | 1 | 0 | 0 | 1 | 0 | 1 | 0 | 0 |
| 21 | NR8383 | ELISA | V | 0 | 1 | 0 | 1 | 0 | 0 | 1 | 0 | 1 | 0 | 0 |
| 22 | NR8383 | ELISA | V | 0 | 1 | 0 | 1 | 0 | 0 | 1 | 0 | 1 | 0 | 0 |
| 23 | THP-1 | ELISA | T | 1 | 0 | 0 | 1 | 0 | 1 | 0 | 0 | 0 | 0 | 1 |
| 24 | RAW 264.7 | ELISA | T | 1 | 0 | 0 | 1 | 0 | 1 | 0 | 0 | 0 | 1 | 0 |
| 25 | RAW 264.7 | ELISA | T | 1 | 0 | 0 | 1 | 0 | 1 | 0 | 0 | 0 | 1 | 0 |
| 26 | Monocyte-derived macrophages | ELISA | T | 0 | 1 | 0 | 1 | 0 | 1 | 0 | 0 | 0 | 0 | 1 |
| 27 | Monocyte-derived macrophages | ELISA | T | 0 | 1 | 0 | 1 | 1 | 1 | 0 | 0 | 0 | 0 | 1 |
| 28 | Monocyte-derived macrophages | RT-PCR | T | 0 | 1 | 0 | 1 | 0 | 1 | 0 | 0 | 0 | 0 | 1 |
| 29 | Alveolar macrophages | Radioimmuno assay | T | 0 | 1 | 0 | 1 | 0 | 0 | 1 | 0 | 1 | 0 | 0 |
| 30 | Alveolar macrophages | Radioimmuno assay | T | 0 | 1 | 0 | 1 | 0 | 0 | 1 | 0 | 1 | 0 | 0 |
| 31 | Alveolar macrophages | Radioimmuno assay | T | 0 | 1 | 0 | 1 | 0 | 0 | 1 | 0 | 1 | 0 | 0 |

*Table A2_2. Nanoform characteristics of samples used in development and validation of method A-2 (T - training set, V - validation set).*

| ID | Split | Spherical | Elongated | Inconclusive | Minimum particle size [nm] | Mean particle size [nm] | Maximum particle size [nm] | Crystal structure - anatase | Crystal structure - rutile | Surface chemistry |
|---|---|---|---|---|---|---|---|---|---|---|
| 0 | T | 1 | 0 | 0 | 10.0 | 10.0 | 10.0 | 1.00 | 0.00 | 0 |
| 1 | T | 1 | 0 | 0 | 20.0 | 20.0 | 20.0 | 0.81 | 0.19 | 0 |
| 2 | T | 1 | 0 | 0 | 100.0 | 100.0 | 100.0 | 1.00 | 0.00 | 0 |
| 3 | T | 1 | 0 | 0 | 26.0 | 34.0 | 56.0 | 0.8 | 0.20 | 0 |
| 4 | V | 1 | 0 | 0 | 7.0 | 7.0 | 7.0 | 1.00 | 0.00 | 0 |
| 5 | T | 1 | 0 | 0 | 6.1 | 7.0 | 7.9 | 1.00 | 0.00 | 0 |
| 6 | T | 0 | 1 | 0 | 20.8 | 33.3 | 45.8 | 1.00 | 0.00 | 0 |
| 7 | T | 1 | 0 | 0 | 13.4 | 43.8 | 90.6 | 0.79 | 0.21 | 0 |
| 8 | T | 1 | 0 | 0 | 13.4 | 43.8 | 90.6 | 0.79 | 0.21 | 0 |
| 9 | T | 1 | 0 | 0 | 29.2 | 69.1 | 109.0 | 0.80 | 0.20 | 0 |
| 10 | T | 0 | 0 | 1 | 10.0 | 10.0 | 10.0 | 1.00 | 0.00 | 0 |
| 11 | V | 0 | 0 | 1 | 10.0 | 10.0 | 10.0 | 1.00 | 0.00 | 0 |
| 12 | T | 0 | 0 | 1 | 21.0 | 21.0 | 21.0 | 0.80 | 0.20 | 0 |
| 13 | T | 0 | 0 | 1 | 53.0 | 110.0 | 167.0 | 1.00 | 0.00 | 0 |
| 14 | T | 0 | 0 | 1 | 5.3 | 6.0 | 6.7 | 1.00 | 0.00 | 0 |
| is | T | 0 | 0 | 1 | 53.0 | 110.0 | 167.0 | 1.00 | 0.00 | 0 |
| 16 | V | 0 | 0 | 1 | 5.3 | 6.0 | 6.7 | 1.00 | 0.00 | 0 |
| 17 | T | 0 | 0 | 1 | 10.0 | 10.0 | 10.0 | 1.00 | 0.00 | 0 |
| 18 | T | 0 | 0 | 1 | 50.0 | 50.0 | 50.0 | 1.00 | 0.00 | 0 |
| 19 | V | 0 | 0 | 1 | 12.0 | 15.0 | 18.0 | 0.80 | 0.20 | 0 |
| 20 | V | 0 | 0 | 1 | 10.0 | 10.0 | 10.0 | 1.00 | 0.00 | 0 |
| 21 | V | 0 | 0 | 1 | 12.0 | 13.5 | 15.0 | 1.00 | 0.00 | 0 |
| 22 | V | 0 | 0 | 1 | 16.0 | 16.5 | 17.0 | 1.00 | 0.00 | 0 |
| 23 | T | 0 | 0 | 1 | 17.0 | 17.0 | 17.0 | 1.00 | 0.00 | 0 |

(continued)

| ID | Split | Spherical | Elongated | Inconclusive | Minimum particle size [nm] | Mean particle size [nm] | Maximum particle size [nm] | Crystal structure - anatase | Crystal structure - rutile | Surface chemistry |
|---|---|---|---|---|---|---|---|---|---|---|
| 24 | T | 0 | 1 | 0 | 40.0 | 40.0 | 40.0 | 0.00 | 1.00 | 1 |
| 25 | T | 0 | 0 | 1 | 30.0 | 35.0 | 40.0 | 0.00 | 1.00 | 0 |
| 26 | T | 0 | 1 | 0 | 40.0 | 40.0 | 40.0 | 0.00 | 1.00 | 1 |
| 27 | T | 0 | 0 | 1 | 30.0 | 35.0 | 40.0 | 0.00 | 1.00 | 0 |
| 28 | T | 0 | 1 | 0 | 40.0 | 40.0 | 40.0 | 0.00 | 1.00 | 1 |
| 29 | T | 0 | 0 | 1 | 5.0 | 5.0 | 5.0 | 1.00 | 0.00 | 0 |
| 30 | T | 0 | 0 | 1 | 21.0 | 21.0 | 21.0 | 0.80 | 0.20 | 0 |
| 31 | T | 0 | 0 | 1 | 50.0 | 50.0 | 50.0 | 0.00 | 1.00 | 0 |

*Table A2_3. Concentrations of samples used in development and validation of method A-2 (T - training set, V - validation set).*

| ID | Split | Concentration [μg/mL] |
|---|---|---|
| 0 | T | 10.00 |
| 1 | T | 100.00 |
| 2 | T | 100.00 |
| 3 | T | 3.20 |
| 4 | V | 32.00 |
| 5 | T | 16.00 |
| 6 | T | 16.00 |
| 7 | T | 40.00 |
| 8 | T | 40.00 |
| 9 | T | 304.00 |
| 10 | T | 100.00 |
| 11 | V | 10.00 |
| 12 | T | 0.50 |
| 13 | T | 100.00 |
| 14 | T | 100.00 |
| 1 5 | T | 10.00 |
| 16 | V | 10.00 |
| 17 | T | 1.00 |
| 18 | T | 1.00 |
| 19 | V | 12.50 |
| 20 | V | 12.50 |
| 21 | V | 12.50 |
| 22 | V | 12.50 |
| 23 | T | 256.00 |
| 24 | T | 100.00 |
| 25 | T | 100.00 |
| 26 | T | 100.00 |
| 27 | T | 300.00 |
| 28 | T | 300.00 |
| 29 | T | 18.75 |
| 30 | T | 300.00 |
| 31 | T | 300.00 |

*Table A2_4. Proinflammatory induction of TNF-α of samples used in development and validation of method A-2 (1 - positive, 0 - negative response) (T - training set, V - validation set).*

| ID | Split | Proinflammation effect |
|---|---|---|
| 0 | T | 1 |

(continued)

| ID | Split | Proinflammation effect |
|---|---|---|
| 1 | T | 1 |
| 2 | T | 1 |
| 3 | T | 1 |
| 4 | V | 0 |
| 5 | T | 1 |
| 6 | T | 1 |
| 7 | T | 0 |
| 8 | T | 0 |
| 9 | T | 1 |
| 10 | T | 1 |
| 11 | V | 1 |
| 12 | T | 1 |
| 13 | T | 0 |
| 14 | T | 0 |
| 15 | T | 1 |
| 16 | V | 1 |
| 17 | T | 1 |
| 18 | T | 1 |
| 19 | V | 1 |
| 20 | V | 1 |
| 21 | V | 0 |
| 22 | V | 1 |
| 23 | T | 1 |
| 24 | T | 1 |
| 25 | T | 0 |
| 26 | T | 1 |
| 27 | T | 0 |
| 28 | T | 1 |
| 29 | T | 1 |
| 30 | T | 1 |
| 31 | T | 1 |

*Table A2_5. Scores values of samples used in development and validation of method A-2 (T - training set, V - validation set).*

| ID | Split | Nanoform characteristic | | Cell line characteristic | |
|---|---|---|---|---|---|
| | | PC1 | PC2 | PC1 | PC2 |
| 0 | T | -1.4457 | -1.01245 | -2.57294 | -1.0315 |
| 1 | T | -0.8694 | -0.52217 | -2.57294 | -1.0315 |

(continued)

| ID | Split | Nanoform characteristic | | Cell line characteristic | |
|---|---|---|---|---|---|
| | | PC1 | PC2 | PC1 | PC2 |
| 2 | T | -0.32384 | 4.052335 | -2.57294 | -1.0315 |
| 3 | T | -0.74177 | 0.35727 | 3.366016 | -0.95798 |
| 4 | V | -1.4831 | -1.18128 | 3.366016 | -0.95798 |
| 5 | T | -1.49138 | -1.1907 | 3.366016 | -0.95798 |
| 6 | T | 0.3282 | -0.38544 | 3.366016 | -0.95798 |
| 7 | T | -0.80155 | 0.701189 | 2.086279 | -2.16548 |
| 8 | T | -0.80155 | 0.701189 | 3.366016 | -0.95798 |
| 9 | T | -0.60094 | 1.819482 | 0.183621 | 3.805697 |
| 10 | T | -1.36639 | -1.55598 | -2.57294 | -1.0315 |
| 11 | V | -1.36639 | -1.55598 | -0.7904 | 0.970065 |
| 12 | T | -0.75386 | -1.01324 | -2.57294 | -1.0315 |
| 13 | T | -0.64466 | 3.474901 | 3.366016 | -0.95798 |
| 14 | T | -1.4227 | -1.78841 | 3.366016 | -0.95798 |
| 15 | T | -0.64466 | 3.474901 | -2.57294 | -1.0315 |
| 16 | V | -1.4227 | -1.78841 | -2.57294 | -1.0315 |
| 17 | T | -1.36639 | -1.55598 | -0.7904 | 0.970065 |
| 18 | T | -0.86779 | 0.695031 | -0.7904 | 0.970065 |
| 19 | V | -0.85627 | -1.3823 | 0.183621 | 3.805697 |
| 20 | V | -1.36639 | -1.55598 | 0.183621 | 3.805697 |
| 21 | V | -1.33657 | -1.37472 | 0.183621 | 3.805697 |
| 22 | V | -1.28997 | -1.19543 | 0.183621 | 3.805697 |
| 23 | T | -1.27914 | -1.16206 | -1.17294 | -1.15502 |
| 24 | T | 4.404732 | -0.46238 | -2.57294 | -1.0315 |
| 25 | T | 1.276295 | -0.58286 | -2.57294 | -1.0315 |
| 26 | T | 4.404732 | -0.46238 | 0.1068 | 0.052481 |
| 27 | T | 1.276295 | -0.58286 | 0.1068 | 0.052481 |
| 28 | T | 4.404732 | -0.46238 | 0.1068 | 0.052481 |
| 29 | T | -1.42872 | -1.83736 | 0.183621 | 3.805697 |
| 30 | T | -0.75386 | -1.01324 | 0.183621 | 3.805697 |
| 31 | T | 1.509305 | 0.313604 | 0.183621 | 3.805697 |

Table B1_1. Cell line characteristics of samples used in development and validation of method B-1 (T – training set, V – validation set).

| ID | Cell line | Test assay | Split | Human | Mouse | Rat | Epithelium lung | Macrophage lung | Macrophage monocyte | Dendritic monocyte | Epithelium cervix | Epithelium liver | Neuron brain | Microglial brain | Macrophage bone marrow | Endothelium umbilical vein | Mesoderm | Endoderm | Ectoderm | Normal | Cancer |
|---|---|---|---|---|---|---|---|---|---|---|---|---|---|---|---|---|---|---|---|---|---|
| 0 | HUVEC | ELISA | T | 1 | 0 | 0 | 0 | 0 | 0 | 0 | 0 | 0 | 0 | 0 | 0 | 1 | 1 | 0 | 0 | 1 | 0 |
| 1 | HeLa | RT-PCR | T | 1 | 0 | 0 | 0 | 0 | 0 | 0 | 1 | 0 | 0 | 0 | 0 | 0 | 1 | 0 | 0 | 0 | 1 |
| 2 | THP-1 | RT-PCR | V | 1 | 0 | 0 | 0 | 0 | 1 | 0 | 0 | 0 | 0 | 0 | 0 | 0 | 1 | 0 | 0 | 0 | 1 |
| 3 | HepG2 | RT-PCR | T | 1 | 0 | 0 | 0 | 0 | 0 | 0 | 0 | 1 | 0 | 0 | 0 | 0 | 0 | 1 | 0 | 0 | 1 |
| 4 | A549 | ELISA | T | 1 | 0 | 0 | 1 | 0 | 0 | 0 | 0 | 0 | 0 | 0 | 0 | 0 | 0 | 1 | 0 | 0 | 1 |
| 5 | BEAS-2B | ELISA | T | 1 | 0 | 0 | 1 | 0 | 0 | 0 | 0 | 0 | 0 | 0 | 0 | 0 | 0 | 1 | 0 | 1 | 0 |
| 6 | TT1 | ELISA | T | 1 | 0 | 0 | 1 | 0 | 0 | 0 | 0 | 0 | 0 | 0 | 0 | 0 | 0 | 1 | 0 | 1 | 0 |
| 7 | TT1 | ELISA | T | 1 | 0 | 0 | 1 | 0 | 0 | 0 | 0 | 0 | 0 | 0 | 0 | 0 | 0 | 1 | 0 | 1 | 0 |
| 8 | A549 | ELISA | V | 1 | 0 | 0 | 1 | 0 | 0 | 0 | 0 | 0 | 0 | 0 | 0 | 0 | 0 | 1 | 0 | 0 | 1 |
| 9 | A549 | ELISA | T | 1 | 0 | 0 | 1 | 0 | 0 | 0 | 0 | 0 | 0 | 0 | 0 | 0 | 0 | 1 | 0 | 0 | 1 |
| 10 | BEAS-2B | ELISA | V | 1 | 0 | 0 | 1 | 0 | 0 | 0 | 0 | 0 | 0 | 0 | 0 | 0 | 0 | 1 | 0 | 1 | 0 |
| 11 | BEAS-2B | ELISA | T | 1 | 0 | 0 | 1 | 0 | 0 | 0 | 0 | 0 | 0 | 0 | 0 | 0 | 0 | 1 | 0 | 1 | 0 |
| 12 | A549 | RT-PCR | T | 1 | 0 | 0 | 1 | 0 | 0 | 0 | 0 | 0 | 0 | 0 | 0 | 0 | 0 | 1 | 0 | 0 | 1 |
| 13 | RAW 264.7 | RT-PCR | T | 0 | 1 | 0 | 0 | 0 | 1 | 0 | 0 | 0 | 0 | 0 | 0 | 0 | 1 | 0 | 0 | 0 | 1 |
| 14 | N2a | ELISA | T | 0 | 1 | 0 | 0 | 0 | 0 | 0 | 0 | 0 | 1 | 0 | 0 | 0 | 0 | 0 | 1 | 0 | 1 |
| 15 | BV-2 | ELISA | T | 0 | 1 | 0 | 0 | 0 | 0 | 0 | 0 | 0 | 0 | 1 | 0 | 0 | 1 | 0 | 0 | 0 | 1 |
| 16 | MH-S | Milliplex Map Kit | T | 0 | 1 | 0 | 0 | 1 | 0 | 0 | 0 | 0 | 0 | 0 | 0 | 0 | 1 | 0 | 0 | 1 | 0 |
| 17 | BEAS-2B | ELISA | T | 1 | 0 | 0 | 1 | 0 | 0 | 0 | 0 | 0 | 0 | 0 | 0 | 0 | 0 | 1 | 0 | 1 | 0 |

| | | | | | | | | | | | | | | | | | | | | |
|---|---|---|---|---|---|---|---|---|---|---|---|---|---|---|---|---|---|---|---|---|
| 18 | BEAS-2B | ELISA | V | 1 | 0 | 0 | 1 | 0 | 0 | 0 | 0 | 0 | 0 | 0 | 0 | 0 | 1 | 0 | 1 | 0 |
| 19 | RAW 264.7 | ELISA | T | 0 | 1 | 0 | 0 | 0 | 1 | 0 | 0 | 0 | 0 | 0 | 0 | 1 | 0 | 0 | 0 | 1 |
| 20 | BMDMs | RT-PCR | T | 0 | 1 | 0 | 0 | 0 | 0 | 0 | 0 | 0 | 0 | 1 | 0 | 1 | 0 | 0 | 1 | 0 |
| 21 | BMDMs | RT-PCR | T | 0 | 1 | 0 | 0 | 0 | 0 | 0 | 0 | 0 | 0 | 1 | 0 | 1 | 0 | 0 | 1 | 0 |
| 22 | BMDMs | RT-PCR | T | 0 | 1 | 0 | 0 | 0 | 0 | 0 | 0 | 0 | 0 | 1 | 0 | 1 | 0 | 0 | 1 | 0 |
| 23 | BMDMs | RT-PCR | T | 0 | 1 | 0 | 0 | 0 | 0 | 0 | 0 | 0 | 0 | 1 | 0 | 1 | 0 | 0 | 1 | 0 |
| 24 | BMDMs | RT-PCR | V | 0 | 1 | 0 | 0 | 0 | 0 | 0 | 0 | 0 | 0 | 1 | 0 | 1 | 0 | 0 | 1 | 0 |
| 25 | BMDMs | RT-PCR | V | 0 | 1 | 0 | 0 | 0 | 0 | 0 | 0 | 0 | 0 | 1 | 0 | 1 | 0 | 0 | 1 | 0 |
| 26 | 16HBE14o- | ELISA | T | 1 | 0 | 0 | 1 | 0 | 0 | 0 | 0 | 0 | 0 | 0 | 0 | 0 | 1 | 0 | 1 | 0 |
| 27 | DCs | Bio-Plex system | T | 1 | 0 | 0 | 0 | 0 | 0 | 1 | 0 | 0 | 0 | 0 | 0 | 1 | 0 | 0 | 1 | 0 |
| 28 | alveolar macrophages | radio-immuno-assay | T | 0 | 0 | 1 | 0 | 1 | 0 | 0 | 0 | 0 | 0 | 0 | 0 | 1 | 0 | 0 | 1 | 0 |
| 29 | alveolar macrophages | radio-immuno-assay | T | 0 | 0 | 1 | 0 | 1 | 0 | 0 | 0 | 0 | 0 | 0 | 0 | 1 | 0 | 0 | 1 | 0 |

BMDMs- Bone marrow derived macrophages

TT1 cells - alveolar type-I-like epithelial cells

N2a - (Neuro2a) neuroblastoma cells

DCs - human monocyte–derived Dendritic Cells

Table B1_2. Nanoform characteristics of samples used in development and validation of method B-1 (T - training set, V - validation set).

| ID | Split | Spherical | Irregular | Inconclusive | Minimum particle size [nm] | Mean particle size [nm] | Maximum particle size [nm] | Crystal structure - anatase | Crystal structure - rutile | Surface chemistry |
|---|---|---|---|---|---|---|---|---|---|---|
| 0 | T | 0 | 0 | 1 | 4.0 | 7.0 | 8.0 | 0.00 | 1.00 | 0 |
| 1 | T | 0 | 0 | 1 | 49.6 | 49.6 | 49.6 | 0.80 | 0.20 | 1 |
| 2 | V | 0 | 0 | 1 | 49.6 | 49.6 | 49.6 | 0.80 | 0.20 | 1 |
| 3 | T | 0 | 0 | 1 | 49.6 | 49.6 | 49.6 | 0.80 | 0.20 | 1 |
| 4 | T | 1 | 0 | 0 | 26.8 | 43.8 | 60.8 | 0.79 | 0.21 | 0 |
| 5 | T | 1 | 0 | 0 | 26.8 | 43.8 | 60.8 | 0.79 | 0.21 | 0 |
| 6 | T | 1 | 0 | 0 | 6.0 | 8.0 | 10.0 | 1.00 | 0.00 | 0 |
| 7 | T | 1 | 0 | 0 | 5.0 | 8.5 | 12.0 | 0.00 | 1.00 | 0 |
| 8 | V | 1 | 0 | 0 | 26.8 | 43.8 | 60.8 | 0.79 | 0.21 | 0 |
| 9 | T | 0 | 1 | 0 | 23.9 | 76.6 | 129.3 | 0.19 | 0.81 | 0 |
| 10 | V | 1 | 0 | 0 | 26.8 | 43.8 | 60.8 | 0.79 | 0.21 | 0 |
| 11 | T | 0 | 1 | 0 | 23.9 | 76.6 | 129.3 | 0.19 | 0.81 | 0 |
| 12 | T | 1 | 0 | 0 | 23.0 | 23.0 | 23.0 | 1.00 | 1.00 | 0 |
| 13 | T | 1 | 0 | 0 | 23.0 | 23.0 | 23.0 | 1.00 | 1.00 | 0 |
| 14 | T | 0 | 0 | 1 | 5.8 | 5.8 | 5.8 | 1.00 | 0.00 | 0 |
| 15 | T | 0 | 0 | 1 | 5.8 | 5.8 | 5.8 | 1.00 | 0.00 | 0 |
| 16 | T | 0 | 0 | 1 | 10.0 | 10.0 | 10.0 | 1.00 | 0.00 | 0 |
| 17 | T | 0 | 0 | 1 | 53.0 | 110.0 | 167.0 | 1.00 | 0.00 | 0 |
| 18 | V | 0 | 0 | 1 | 5.3 | 6.00 | 6.7 | 1.00 | 0.00 | 0 |
| 19 | T | 0 | 0 | 1 | 53.0 | 110.0 | 167.0 | 1.00 | 0.00 | 0 |
| 20 | T | 0 | 0 | 1 | 10.0 | 10.0 | 10.0 | 1.00 | 0.00 | 0 |
| 21 | T | 0 | 0 | 1 | 50.0 | 50.0 | 50.0 | 1.00 | 0.00 | 0 |
| 22 | T | 0 | 0 | 1 | 12.0 | 15.0 | 18.0 | 0.80 | 0.20 | 0 |
| 23 | T | 0 | 0 | 1 | 10.0 | 10.0 | 10.0 | 1.00 | 0.00 | 0 |

(continued)

| ID | Split | Spherical | Irregular | Inconclusive | Minimum particle size [nm] | Mean particle size [nm] | Maximum particle size [nm] | Crystal structure - anatase | Crystal structure - rutile | Surface chemistry |
|----|-------|-----------|-----------|--------------|---------------------------|-------------------------|----------------------------|----------------------------|---------------------------|-------------------|
| 24 | V | 0 | 0 | 1 | 12.0 | 13.5 | 15.0 | 1.00 | 0.00 | 0 |
| 25 | V | 0 | 0 | 1 | 16.0 | 16.5 | 17.0 | 1.00 | 0.00 | 0 |
| 26 | T | 0 | 0 | 1 | 15.0 | 15.0 | 15.0 | 1.00 | 0.00 | 0 |
| 27 | T | 0 | 0 | 1 | 7.0 | 8.5 | 10.0 | 1.00 | 0.00 | 0 |
| 28 | T | 0 | 0 | 1 | 5.0 | 5.0 | 5.0 | 1.00 | 0.00 | 0 |
| 29 | T | 0 | 0 | 1 | 50.0 | 50.0 | 50.0 | 0.00 | 1.00 | 0 |

*Table B1_3. Proinflammatory induction of IL-6 expressed with NOEC class for samples used in development and validation of method B-1 (T - training set, V - validation set).*

| ID | Split | NOEC class |
|---|---|---|
| 0 | T | CLASS II |
| 1 | T | CLASS II |
| 2 | V | CLASS II |
| 3 | T | CLASS II |
| 4 | T | CLASS I |
| 5 | T | CLASS II |
| 6 | T | CLASS I |
| 7 | T | CLASS I |
| 8 | V | CLASS I |
| 9 | T | CLASS I |
| 10 | V | CLASS II |
| 11 | T | CLASS II |
| 12 | T | CLASS III |
| 13 | T | CLASS III |
| 14 | T | CLASS II |
| 15 | T | CLASS II |
| 16 | T | CLASS I |
| 17 | T | CLASS II |
| 18 | V | CLASS I |
| 19 | T | CLASS I |
| 20 | T | CLASS I |
| 21 | T | CLASS I |
| 22 | T | CLASS III |
| 23 | T | CLASS III |
| 24 | V | CLASS III |
| 25 | V | CLASS III |
| 26 | T | CLASS II |
| 27 | T | CLASS I |
| 28 | T | CLASS II |
| 29 | T | CLASS III |

*Table B1_4. Scores values of samples used in development and validation of method B-1 (T - training set, V - validation set).*

| ID | Split | Nanoform characteristics | | Cell line characteristics | | |
|---|---|---|---|---|---|---|
| | | PC1 | PC2 | PC1 | PC2 | PC3 |
| 0 | T | 0.036995 | 1.84042 | -0.21486 | -1.52114 | -0.55212 |
| 1 | T | 0.341242 | -2.37721 | -0.01471 | 1.506397 | -2.26288 |

51

(continued)

| ID | Split | Nanoform characteristics | | Cell line characteristics | | |
|---|---|---|---|---|---|---|
| | | PC1 | PC2 | PC1 | PC2 | PC3 |
| 2 | V | 0.341242 | -2.37721 | -0.51217 | 1.78992 | -2.05998 |
| 3 | T | 0.341242 | -2.37721 | 2.204682 | 1.838055 | -1.06061 |
| 4 | T | 0.575611 | 0.897973 | 2.553747 | 1.120093 | -0.33769 |
| 5 | T | 0.575611 | 0.897973 | 2.398602 | -0.92387 | 0.4679 |
| 6 | T | -1.16849 | 1.652521 | 2.398602 | -0.92387 | 0.4679 |
| 7 | T | 0.824911 | 3.550554 | 2.398602 | -0.92387 | 0.4679 |
| 8 | V | 0.575611 | 0.897973 | 2.553747 | 1.120093 | -0.33769 |
| 9 | T | 4.100903 | 0.759281 | 2.553747 | 1.120093 | -0.33769 |
| 10 | V | 0.575611 | 0.897973 | 2.398602 | -0.92387 | 0.4679 |
| 11 | T | 4.100903 | 0.759281 | 2.398602 | -0.92387 | 0.4679 |
| 12 | T | 0.450814 | 1.973436 | 2.553747 | 1.120093 | -0.33769 |
| 13 | T | 0.450814 | 1.973436 | -2.13034 | 2.275673 | -1.79768 |
| 14 | T | -1.95401 | -0.06731 | -1.46325 | 4.98237 | 5.169435 |
| 15 | T | -1.95401 | -0.06731 | -2.10225 | 2.225897 | -1.70586 |
| 16 | T | -1.78337 | -0.2327 | -2.30665 | -1.30298 | 0.531893 |
| 17 | T | 1.795321 | -3.19057 | 2.398602 | -0.92387 | 0.4679 |
| 18 | V | -1.95182 | -0.06316 | 2.398602 | -0.92387 | 0.4679 |
| 19 | T | 1.795321 | -3.19057 | -2.13034 | 2.275673 | -1.79768 |
| 20 | T | -1.78337 | -0.2327 | -2.40713 | -0.864 | 0.005893 |
| 21 | T | -0.15828 | -1.80782 | -2.40713 | -0.864 | 0.005893 |
| 22 | T | -1.20854 | 0.000371 | -2.40713 | -0.864 | 0.005893 |
| 23 | T | -1.78337 | -0.2327 | -2.40713 | -0.864 | 0.005893 |
| 24 | V | -1.65391 | -0.34474 | -2.40713 | -0.864 | 0.005893 |
| 25 | V | -1.52354 | -0.48006 | -2.40713 | -0.864 | 0.005893 |
| 26 | T | -1.58023 | -0.42959 | 2.398602 | -0.92387 | 0.4679 |
| 27 | T | -1.85705 | -0.14785 | -0.21486 | -1.52114 | -0.55212 |
| 28 | T | -1.98651 | -0.03581 | -2.02587 | -2.55992 | 1.104861 |
| 29 | T | 1.827543 | 0.084117 | -2.02587 | -2.55992 | 1.104861 |

Table B2_1. Cell line characteristics of samples used in development and validation of method B-2 (T – training set, V – validation set).

| ID | Cell line | Test assay | Split | Human | Mouse | Rat | Epithelium lung | Macrophage lung alveolar | Macrophage monocyte | Macrophage bone marrow | Mesoderm | Endoderm | Normal | Cancer |
|---|---|---|---|---|---|---|---|---|---|---|---|---|---|---|
| 0 | RAW264.7 | ELISA | T | 0 | 1 | 0 | 0 | 0 | 1 | 0 | 1 | 0 | 0 | 1 |
| 1 | RAW264.7 | ELISA | T | 0 | 1 | 0 | 0 | 0 | 1 | 0 | 1 | 0 | 0 | 1 |
| 2 | BEAS-2B | ELISA | T | 1 | 0 | 0 | 1 | 0 | 0 | 0 | 0 | 1 | 1 | 0 |
| 3 | BEAS-2B | ELISA | V | 1 | 0 | 0 | 1 | 0 | 0 | 0 | 0 | 1 | 1 | 0 |
| 4 | BEAS-2B | ELISA | T | 1 | 0 | 0 | 1 | 0 | 0 | 0 | 0 | 1 | 1 | 0 |
| 5 | A549 | ELISA | T | 1 | 0 | 0 | 1 | 0 | 0 | 0 | 0 | 1 | 0 | 1 |
| 6 | BEAS-2B | ELISA | V | 1 | 0 | 0 | 1 | 0 | 0 | 0 | 0 | 1 | 1 | 0 |
| 7 | NR8383 | ELISA | T | 0 | 0 | 1 | 0 | 1 | 0 | 0 | 1 | 0 | 1 | 0 |
| 8 | RAW 264.7 | RT-PCR | T | 0 | 1 | 0 | 0 | 0 | 1 | 0 | 1 | 0 | 0 | 1 |
| 10 | BEAS-2B | ELISA | V | 1 | 0 | 0 | 1 | 0 | 0 | 0 | 0 | 1 | 1 | 0 |
| 11 | BEAS-2B | ELISA | V | 1 | 0 | 0 | 1 | 0 | 0 | 0 | 0 | 1 | 1 | 0 |
| 12 | BMDMs | RT-PCR | T | 0 | 1 | 0 | 0 | 0 | 0 | 1 | 1 | 0 | 1 | 0 |
| 13 | BMDMs | RT-PCR | V | 0 | 1 | 0 | 0 | 0 | 0 | 1 | 1 | 0 | 1 | 0 |
| 14 | NR8383 | ELISA | T | 0 | 0 | 1 | 0 | 1 | 0 | 0 | 1 | 0 | 1 | 0 |
| 15 | NR8383 | ELISA | V | 0 | 0 | 1 | 0 | 1 | 0 | 0 | 1 | 0 | 1 | 0 |
| 16 | NR8383 | ELISA | T | 0 | 0 | 1 | 0 | 1 | 0 | 0 | 1 | 0 | 1 | 0 |
| 17 | NR8383 | ELISA | T | 0 | 0 | 1 | 0 | 1 | 0 | 0 | 1 | 0 | 1 | 0 |
| 18 | RAW 264.7 | ELISA | T | 0 | 1 | 0 | 0 | 0 | 1 | 0 | 1 | 0 | 0 | 1 |
| 19 | RAW 264.7 | ELISA | T | 0 | 1 | 0 | 0 | 0 | 1 | 0 | 1 | 0 | 0 | 1 |
| 20 | Monocyte–derived | ELISA | T | 1 | 0 | 0 | 0 | 0 | 1 | 0 | 1 | 0 | 1 | 0 |

| | | | | | | | | | | | | | | |
|---|---|---|---|---|---|---|---|---|---|---|---|---|---|---|
| | macrophages | | | | | | | | | | | | | |
| 21 | Monocyte–derived macrophages | ELISA | T | 1 | 0 | 0 | 0 | 0 | 1 | 0 | 1 | 0 | 1 | 0 |
| 22 | Alveolar macrophages | radioimmunoassay | V | 0 | 0 | 1 | 0 | 1 | 0 | 0 | 1 | 0 | 1 | 0 |
| 23 | Alveolar macrophages | radioimmunoassay | T | 0 | 0 | 1 | 0 | 1 | 0 | 0 | 1 | 0 | 1 | 0 |

BMDMs    -Bone    marrow    derived    macrophages

EP 4 376 011 A1

*Table B2_2. Nanoform characteristics of samples used in development and validation of method B-2 (T - training set, V - validation set).*

| ID | Split | Spherical | Elongated | Inconclusive | Minimum particle size [nm] | Mean particle size [nm] | Maximum particle size [nm] | Crystal structure - anatase | Crystal structure - rutile | Surface chemistry |
|---|---|---|---|---|---|---|---|---|---|---|
| 0 | T | 1 | 0 | 0 | 20.0 | 20.0 | 20.0 | 0.81 | 0.19 | 0 |
| 1 | T | 1 | 0 | 0 | 100.0 | 100.0 | 100.0 | 1.00 | 0.00 | 0 |
| 2 | T | 1 | 0 | 0 | 7.0 | 7.0 | 7.0 | 1.00 | 0.00 | 0 |
| 3 | V | 1 | 0 | 0 | 6.1 | 7.0 | 7.9 | 1.00 | 0.00 | 0 |
| 4 | T | 0 | 1 | 0 | 20.8 | 33.3 | 45.8 | 1.00 | 0.00 | 0 |
| 5 | T | 1 | 0 | 0 | 13.4 | 43.8 | 90.6 | 0.79 | 0.21 | 0 |
| 6 | V | 1 | 0 | 0 | 13.4 | 43.8 | 90.6 | 0.79 | 0.21 | 0 |
| 7 | T | 1 | 0 | 0 | 29.2 | 69.1 | 109.0 | 0.80 | 0.20 | 0 |
| 8 | T | 0 | 0 | 1 | 10.0 | 10.0 | 10.0 | 1.00 | 0.00 | 0 |
| 10 | V | 0 | 0 | 1 | 53.0 | 110.0 | 167.0 | 1.00 | 0.00 | 0 |
| 11 | V | 0 | 0 | 1 | 5.3 | 6.0 | 6.7 | 1.00 | 0.00 | 0 |
| 12 | T | 0 | 0 | 1 | 10.0 | 10.0 | 10.0 | 1.00 | 0.00 | 0 |
| 13 | V | 0 | 0 | 1 | 50.0 | 50.0 | 50.0 | 1.00 | 0.00 | 0 |
| 14 | T | 0 | 0 | 1 | 12.0 | 15.0 | 18.0 | 0.80 | 0.20 | 0 |
| is | V | 0 | 0 | 1 | 10.0 | 10.0 | 10.0 | 1.00 | 0.00 | 0 |
| 16 | T | 0 | 0 | 1 | 12.0 | 13.5 | 15.0 | 1.00 | 0.00 | 0 |
| 17 | T | 0 | 0 | 1 | 16.0 | 16.5 | 17.0 | 1.00 | 0.00 | 0 |
| 18 | T | 0 | 1 | 0 | 40.0 | 40.0 | 40.0 | 0.00 | 1.00 | 1 |
| 19 | T | 0 | 0 | 1 | 30.0 | 35.0 | 40.0 | 0.00 | 1.00 | 0 |
| 20 | T | 0 | 1 | 0 | 40.0 | 40.0 | 40.0 | 0.00 | 1.00 | 1 |
| 21 | T | 0 | 0 | 1 | 30.0 | 35.0 | 40.0 | 0.00 | 1.00 | 0 |
| 22 | V | 0 | 0 | 1 | 21.0 | 21.0 | 21.0 | 0.80 | 0.20 | 0 |
| 23 | T | 0 | 0 | 1 | 50.0 | 50.0 | 50.0 | 0.00 | 1.00 | 0 |

*Table B2_3. Proinflammatory induction of IL-6 expressed with NOEC class for samples used in development and validation of method B-2 (T - training set, V - validation set).*

| ID | Split | NOEC class |
|---|---|---|
| 0 | T | CLASS II |
| 1 | T | CLASS II |
| 2 | T | CLASS II |
| 3 | V | CLASS I |
| 4 | T | CLASS I |
| 5 | T | CLASS II |
| 6 | V | CLASS II |
| 7 | T | CLASS III |
| 8 | T | CLASS I |
| 10 | V | CLASS II |
| 11 | V | CLASS II |
| 12 | T | CLASS I |
| 13 | V | CLASS I |
| 14 | T | CLASS II |
| 15 | V | CLASS II |
| 16 | T | CLASS II |
| 17 | T | CLASS II |
| 18 | T | CLASS II |
| 19 | T | CLASS III |
| 20 | T | CLASS II |
| 21 | T | CLASS III |
| 22 | V | CLASS III |
| 23 | T | CLASS III |

*Table B2_4. Scores values of samples used in development and validation of method B-2 (T - training set, V - validation set).*

| ID | Split | Nanoform characteristic | | Cell line characteristic | |
|---|---|---|---|---|---|
| | | PC1 | PC2 | PC1 | PC2 |
| 0 | T | -2.5486 | -1.37879 | -2.5486 | -1.37879 |
| 1 | T | -2.5486 | -1.37879 | -2.5486 | -1.37879 |
| 2 | T | 3.510871 | -1.20329 | 3.510871 | -1.20329 |
| 3 | V | -1.03004 | -1.29897 | 3.510871 | -1.20329 |
| 4 | T | 3.510871 | -1.20329 | 3.510871 | -1.20329 |
| 5 | T | 2.341055 | -2.55304 | 2.341055 | -2.55304 |
| 6 | V | -1.00392 | 0.616388 | 3.510871 | -1.20329 |
| 7 | T | 0.132601 | 3.121362 | 0.132601 | 3.121362 |
| 8 | T | -2.5486 | -1.37879 | -2.5486 | -1.37879 |

(continued)

| ID | Split | Nanoform characteristic | | Cell line characteristic | |
|---|---|---|---|---|---|
| | | PC1 | PC2 | PC1 | PC2 |
| **10** | V | -0.718 | -2.36428 | 3.510871 | -1.20329 |
| **11** | V | -0.75026 | -2.14276 | -0.91606 | 0.620744 |
| **12** | T | 3.510871 | -1.20329 | 3.510871 | -1.20329 |
| **13** | V | -0.33036 | -1.84971 | 0.132601 | 3.121362 |
| **14** | T | -0.91606 | 0.620744 | -0.91606 | 0.620744 |
| **15** | V | -0.78499 | -1.97111 | 0.132601 | 3.121362 |
| **16** | T | 0.132601 | 3.121362 | 0.132601 | 3.121362 |
| **17** | T | 0.132601 | 3.121362 | 0.132601 | 3.121362 |
| **18** | T | -2.5486 | -1.37879 | -2.5486 | -1.37879 |
| **19** | T | -2.5486 | -1.37879 | -2.5486 | -1.37879 |
| **20** | T | 0.127502 | -0.02468 | 0.127502 | -0.02468 |
| **21** | T | 0.127502 | -0.02468 | 0.127502 | -0.02468 |
| **22** | V | 1.266366 | 0.283198 | 0.132601 | 3.121362 |
| **23** | T | 0.132601 | 3.121362 | 0.132601 | 3.121362 |

**Claims**

1. Qualitative structure-activity relationship (QSAR) method for prediction of cytokine IL-6 induction in mammalian cell lines treated with $TiO_2$ nanoparticles, that can be used as an equivalent of *in vitro* experimental measurement with ELISA, RT-PCR or other test assay dedicated to identification of cytokines expression, comprises of the following steps:

   a) providing a dataset of $TiO_2$ nanoform(s) and cell line(s) characteristics, as well as corresponding concentration(s) at which influence on the IL-6 induction was observed in *in vitro* experimental measurements and used as training and validation sets, Table A1_1 - A1_4;
   b) providing dataset pre-processing through standardization, principal component analysis (PCA) and reduction of a number of principal components (PCs) for the separated matrices of nanoforms and cell lines characteristics, with subsequent reconstruction of the decision tree classifier with inputs obtained for training set: i) matrices of reduced number of PCs describing nanoforms and cell lines characteristics, and ii) vector of corresponding concentrations;
   c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics and concentrations, through: i) adherence to a specific group with binary scale (1 - yes, or 0 - no), ii) a value of a specific feature within the indicated range; the characteristics should be expressed as follows:

      ▪ cell line origin:

         ◦ human [1 or 0]
         ◦ mouse [1 or 0]
         ◦ rat [1 or 0]

      ▪ cell type and organ of origin:

         ◦ epithelium lung [1 or 0]
         ◦ epithelium cervix [1 or 0]

○ macrophage monocyte [1 or 0]
○ epithelium liver [1 or 0]
○ neuron brain [1 or 0]
○ microglia brain [1 or 0]
○ macrophage lung [1 or 0]
○ macrophage bone marrow [1 or 0]
○ endothelium umbilical vein [1 or 0]
○ dendritic monocytes [1 or 0]

■ germ layers the cells originate from:

○ endoderm [1 or 0]
○ ectoderm [1 or 0]
○ mesoderm [1 or 0]

■ biological nature of cell used:

○ normal [1 or 0]
○ cancer [1 or 0]

■ shape:

○ spherical [1 or 0]
○ irregular [1 or 0]
○ inconclusive [1 or 0]

■ size:

○ min - minimum particle size, quantitative factor, derived from microscopic TEM images: 4.0-53.0 [nm]
○ mean - mean particle size, quantitative factor, derived from microscopic TEM images: 5.8-110.0 [nm]
○ max - maximum particle size, quantitative factor, derived from microscopic TEM images: 5.8-167.0 [nm]

■ crystal structure:

○ anatase - quantitative factor defining fraction of anatase form in the material: 0.0-1.0
○ rutile - quantitative factor defining fraction of rutile form in the material: 0.0-1.0

■ surface chemistry - qualitative factor defining presence of a coating on nanomaterial surface, expressed in a binary scale i.e.: 1 - presence of coating, 0 - absence of coating;

wherein the tested $TiO_2$ nanoforms concentrations should be in range 0.08-300.00 [$\mu$g/mL];
d) application of the developed model in the form of decision tree classifier for newly defined samples and prediction of IL-6 induction as positive or negative response based on provided descriptors.

2. Qualitative structure-activity relationship (QSAR) method for prediction of cytokine TNF-$\alpha$ induction in mammalian cell lines treated with $TiO_2$ nanoparticles, that can be used as an equivalent of *in vitro* experimental measurement with ELISA, RT-PCR or other test assay dedicated to identification of cytokines expression, comprises of the following steps:

a) providing a dataset of $TiO_2$ nanoform(s) and cell line(s) characteristics, as well as corresponding concentration(s) at which the TNF-$\alpha$ induction was observed in *in vitro* experiments and used as training and validation sets, Table A2_1 - A2_4;
b) providing dataset pre-processing through standardization, principal component analysis (PCA) and reduction of a number of principal components (PCs) for the separated matrices of nanoforms and cell lines characteristics, with subsequent reconstruction of the k-Nearest Neighbours classifier with inputs obtained for training set: i) matrices of reduced number of PCs describing nanoforms and cell lines characteristics, and ii) vector of corresponding concentrations;
c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics, and concentrations, through: i) adherence to a specific group with binary scale (1 - yes, or 0 - no), ii) a value of a specific feature within the indicated range; the characteristics should be expressed as follows:

- cell line origin:

  ◦ human [1 or 0]
  ◦ mouse [1 or 0]
  ◦ rat [1 or 0]

- cell type and organ of origin:

  ◦ macrophage monocyte [1 or 0]
  ◦ epithelium lung [1 or 0]
  ◦ macrophage lung alveolar [1 or 0]
  ◦ macrophage bone marrow [1 or 0]

- germ layers the cells originate from:

  ◦ endoderm [1 or 0]
  ◦ mesoderm [1 or 0]

- biological nature of cell used:

  ◦ normal [1 or 0]
  ◦ cancer [1 or 0]

- shape:

  ◦ spherical [1 or 0]
  ◦ elongated [1 or 0]
  ◦ inconclusive [1 or 0]

- size:

  ◦ min - minimum particle size, quantitative factor, derived from microscopic TEM images: 5.0-100.0 [nm]
  ◦ mean - mean particle size, quantitative factor, derived from microscopic TEM images: 5.0-110.0 [nm]
  ◦ max - maximum particle size, quantitative factor, derived from microscopic TEM images: 5.0-167.0 [nm]

- crystal structure:

  ◦ anatase - quantitative factor defining fraction of anatase form in the material: 0.0-1.0
  ◦ rutile - quantitative factor defining fraction of rutile form in the material: 0.0-1.0

- surface chemistry - qualitative factor defining presence of a coating on nanomaterial surface, expressed in a binary scale, i.e.: 1 - presence of coating, 0 - absence of coating;

wherein the tested $TiO_2$ nanoforms concentrations should be in range 0.5-304.00 [$\mu$g/mL];
d) application of the developed model in the form of k-Nearest Neighbours classifier for newly defined samples and prediction of TNF-$\alpha$ induction as positive or negative response based on provided descriptors.

3. Qualitative structure-activity relationship (QSAR) method for prediction of cytokine IL-6 NOEC class for mammalian cell lines treated with $TiO_2$ nanoparticles, that can be used as an equivalent of *in vitro* experiments using ELISA, RT-PCR or other test assay dedicated to identification of cytokines expression, comprises of the following steps:

a) providing a dataset of $TiO_2$ nanoform(s) and cell line(s) characteristics, as well as corresponding 'no observed effect concentration' (NOEC) which is the highest concentration potentially used in experiments which should not induce a statistically significant increase in the cytokine IL-6 production, obtained in *in vitro* experiments and used as training and validation sets, Table B1_1 - B1_3, where NOEC is classified into three classes of concentration ranges;

b) providing dataset pre-processing through standardization, principal component analysis (PCA) and reduction of a number of principal components (PCs) for the separated matrices of nanoforms and cell lines characteristics, with subsequent reconstruction of the decision tree classifier with inputs obtained for training set: matrices of reduced number of PCs describing nanoforms and cell lines characteristics;

c) mapping - transformation - of new samples i.e., new, untested TiO$_2$ nanoforms with defined cell line and concentration, into principal components retaining all parameters from the PCA algorithm performed on the training set, where new samples are described with nanoform and cell line characteristics through: i) adherence to a specific group with binary scale (1 - yes, or 0 - no), ii) a value of a specific feature within the indicated range; the characteristics should be expressed as follows:

- cell line origin:

  ○ human [1 or 0]
  ○ mouse [1 or 0]
  ○ rat [1 or 0]

- cell type and organ of origin:

  ○ epithelium lung [1 or 0]
  ○ epithelium cervix [1 or 0]
  ○ macrophage monocyte [1 or 0]
  ○ epithelium liver [1 or 0]
  ○ neuron brain [1 or 0]
  ○ microglia brain [1 or 0]
  ○ macrophage lung [1 or 0]
  ○ macrophage bone marrow [1 or 0]
  ○ endothelium umbilical vein [1 or 0]
  ○ dendritic monocytes [1 or 0]

- germ layers the cells originate from:

  ○ endoderm [1 or 0]
  ○ ectoderm [1 or 0]
  ○ mesoderm [1 or 0]

- biological nature of cell used:

  ○ normal [1 or 0]
  ○ cancer [1 or 0]

- shape:

  ○ spherical [1 or 0]
  ○ irregular [1 or 0]
  ○ inconclusive [1 or 0]

- size:

  ○ min - minimum particle size, quantitative factor, derived from microscopic TEM images: 4.0-53.0 [nm]
  ○ mean - mean particle size, quantitative factor, derived from microscopic TEM images: 5.0-110.0 [nm]
  ○ max - maximum particle size, quantitative factor, derived from microscopic TEM images: 5.0-167.0 [nm]

- crystal structure:

  ○ anatase - quantitative factor defining proportion of anatase form in the material: 0.0-1.0

○ rutile - quantitative factor defining proprtion of rutile form in the material: 0.0-1.0

▪ surface chemistry - qualitative factor defining presence of a coating on nanomaterial surface, expressed in a binary scale, i.e.: 1 - presence of coating, 0 - absence of coating;

d) application of the developed model in the form of decision tree classifier for newly defined samples prediction of IL-6 NOEC class i.e., the highest $TiO_2$ nanoform concentration which does not induce IL-6 in exposed mammalian cells based on provided descriptors.

4. Qualitative structure-activity relationship (QSAR) method for prediction of cytokine TNF-$\alpha$ NOEC class for mammalian cell lines treated with $TiO_2$ nanoparticles, that can be used as an equivalent of *in vitro* experimental measurement with ELISA, RT-PCR or other test assay dedicated to identification of cytokines expression, comprises of the following steps:

a) providing a dataset of $TiO_2$ nanoform(s) and cell line(s) characteristics, as well as corresponding 'no observed effect concentration' (NOEC) which is the highest concentration potentially used in experiments which should not induce a statistically significant increase in the cytokine TNF-$\alpha$ production, obtained in *in vitro* experiments and used as training and validation sets, Table B2_1 - B2_3, where NOEC is classified into three classes of concentration ranges;

b) providing dataset pre-processing through standardization, principal component analysis (PCA) and reduction of a number of principal components (PCs) for the separated matrices of nanoforms and cell lines characteristics, with subsequent reconstruction of the random forest classifier with inputs obtained for training set: matrices of reduced number of PCs describing nanoforms and cell lines characteristics;

c) mapping - transformation - of new samples i.e., new, untested $TiO_2$ nanoforms with defined cell line and concentration, into principal components retaining all parameters from the PCA algorithm performed on the training set, where new samples are described with nanoform and cell line characteristics through: i) adherence to a specific group with binary scale (1 - yes, or 0 - no), ii) a value of a specific feature within the indicated range; the characteristics should be expressed as follows:

▪ cell line origin:

○ human [1 or 0]
○ mouse [1 or 0]
○ rat [1 or 0]

▪ cell type and organ of origin:

○ macrophage monocyte [1 or 0]
○ epithelium lung [1 or 0]
○ macrophage lung alveolar [1 or 0]
○ macrophage bone marrow [1 or 0]

▪ germ layers the cells originate from:

○ endoderm [1 or 0]
○ mesoderm [1 or 0]

▪ biological nature of cell used:

○ normal [1 or 0]
○ cancer [1 or 0]

▪ shape:

○ spherical [1 or 0]
○ elongated [1 or 0]
○ inconclusive [1 or 0]

- size:

  - min - minimum particle size, quantitative factor, derived from microscopic TEM images: 5.3-100.0 [nm]
  - mean - mean particle size, quantitative factor, derived from microscopic TEM images: 6.0-110.0 [nm]
  - max - maximum particle size, quantitative factor, derived from microscopic TEM images: 6.7-167.0 [nm]

- crystal structure:

  - anatase - quantitative factor defining fraction of anatase form in the material: 0.0-1.0
  - rutile - quantitative factor defining fraction of rutile form in the material: 0.0-1.0

- surface chemistry - qualitative factor defining presence of a coating on nanomaterial surface, expressed in a binary scale, i.e.: 1 - presence of coating, 0 - absence of coating;

d) application of the developed model in the form of random forest classifier for newly defined samples and prediction of TNF-$\alpha$ NOEC class, the highest TiO$_2$ nanoform concentration which does not induce TNF-$\alpha$ in exposed mammalian cells based on provided descriptors.

Figure 1. Scheme of approach A (method A-1, A-2)

Figure 2. Scheme of approach B (method B-1, B-2)

Europäisches
Patentamt

European
Patent Office

Office européen
des brevets

# EUROPEAN SEARCH REPORT

**Application Number**

EP 22 20 9238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUANG Y. ET AL: "Quantitative Structure-Activity Relationship Models for Predicting Inflammatory Potential of Metal Oxide Nanoparticles", ENVIRONMENTAL HEALTH PERSPECTIVES, vol. 128, no. 6, 1 June 2020 (2020-06-01), pages 067010-1, XP093046881, US ISSN: 0091-6765, DOI: 10.1289/EHP6508 * the whole document * | 1-4 | INV. G16C20/30 ADD. G16C20/70 |
| A | LI J. ET AL: "Nano-QSAR modeling for predicting the cytotoxicity of metallic and metal oxide nanoparticles: A review", ECOTOXICOLOGY AND ENVIRONMENTAL SAFETY, vol. 243, 1 September 2022 (2022-09-01), page 113955, XP093041761, US ISSN: 0147-6513, DOI: 10.1016/j.ecoenv.2022.113955 * the whole document * | 1-4 | |
| A | BUGLAK A. A. ET AL: "Nano-(Q)SAR for Cytotoxicity Prediction of Engineered Nanomaterials", MOLECULES, vol. 24, no. 24, 11 December 2019 (2019-12-11), page 4537, XP093041650, DE ISSN: 1433-1373, DOI: 10.3390/molecules24244537 * the whole document * | 1-4 | **TECHNICAL FIELDS SEARCHED (IPC)** G16C G06N |

-/--

The present search report has been drawn up for all claims

| Place of search | Date of completion of the search | Examiner |
|---|---|---|
| The Hague | 23 May 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

| | Europäisches<br>Patentamt<br>European<br>Patent Office<br>Office européen<br>des brevets | **EUROPEAN SEARCH REPORT** | **Application Number**<br>**EP 22 20 9238** |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| A | PATEL G. ET AL: "Methods to evaluate the toxicity of engineered nanomaterials for biomedical applications: a review",<br>ENVIRONMENTAL CHEMISTRY LETTERS, SPRINGER INTERNATIONAL PUBLISHING, CHAM,<br>vol. 19, no. 6, 22 July 2021 (2021-07-22),<br>pages 4253-4274, XP037598218,<br>ISSN: 1610-3653, DOI:<br>10.1007/S10311-021-01280-1<br>[retrieved on 2021-07-22]<br>* the whole document * | 1-4 | |
| A | PATHAKOTI K. ET AL: "Using experimental data of Escherichia coli to develop a QSAR model for predicting the photo-induced cytotoxicity of metal oxide nanoparticles",<br>JOURNAL OF PHOTOCHEMISTRY AND PHOTOBIOLOGY B: BIOLOGY, ELSEVIER SCIENCE S.A., BASEL, CH,<br>vol. 130, 4 December 2013 (2013-12-04),<br>pages 234-240, XP028616369,<br>ISSN: 1011-1344, DOI:<br>10.1016/J.JPHOTOBIOL.2013.11.023<br>* the whole document * | 1-4 | TECHNICAL FIELDS<br>SEARCHED (IPC) |

-/--

The present search report has been drawn up for all claims

| Place of search | Date of completion of the search | Examiner |
|---|---|---|
| The Hague | 23 May 2023 | Godzina, Przemyslaw |

**page 2 of 4**

Europäisches
Patentamt

European
Patent Office

Office européen
des brevets

**EUROPEAN SEARCH REPORT**

Application Number

EP 22 20 9238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SANG L. ET AL: "Machine Learning for Evaluating the Cytotoxicity of Mixtures of Nano-TiO2 and Heavy Metals: QSAR Model Apply Random Forest Algorithm after Clustering Analysis", MOLECULES, vol. 27, no. 18, 22 September 2022 (2022-09-22), page 6125, XP093041666, DE ISSN: 1433-1373, DOI: 10.3390/molecules27186125 * the whole document * | 1-4 | |
| A | OKSEL C. ET AL: "(Q)SAR modelling of nanomaterial toxicity: A critical review", PARTICUOLOGY, vol. 21, 5 March 2015 (2015-03-05), pages 1-19, XP029231216, ISSN: 1674-2001, DOI: 10.1016/J.PARTIC.2014.12.001 * the whole document * | 1-4 | |
| A | US 2018/101664 A1 (MU YUNSONG [CN] ET AL) 12 April 2018 (2018-04-12) * the whole document * | 1-4 | TECHNICAL FIELDS SEARCHED (IPC) |

-/--

The present search report has been drawn up for all claims

| Place of search | Date of completion of the search | Examiner |
|---|---|---|
| The Hague | 23 May 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 3 of 4**

Europäisches
Patentamt

European
Patent Office

Office européen
des brevets

## EUROPEAN SEARCH REPORT

**Application Number**

EP 22 20 9238

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WYRZYKOWSKA E. ET AL: "Representing and describing nanomaterials in predictive nanoinformatics", NATURE NANOTECHNOLOGY, vol. 17, no. 9, 18 August 2022 (2022-08-18), pages 924-932, XP093041759, London ISSN: 1748-3387, DOI: 10.1038/s41565-022-01173-6 Retrieved from the Internet: URL:https://www.nature.com/articles/s41565-022-01173-6> * the whole document * ----- | 1-4 | |
| A | WARHEIT D. B. ET AL: "What is the impact of surface modifications and particle size on commercial titanium dioxide particle samples? – A review of in vivo pulmonary and oral toxicity studies – Revised 11-6-2018", TOXICOLOGY LETTERS, vol. 302, 20 November 2018 (2018-11-20), pages 42-59, XP085595116, ISSN: 0378-4274, DOI: 10.1016/J.TOXLET.2018.11.008 * the whole document * ----- | 1-4 | TECHNICAL FIELDS SEARCHED (IPC) |
| T | RACOVITA A. D.: "Titanium Dioxide: Structure, Impact, and Toxicity", INTERNATIONAL JOURNAL OF ENVIRONMENTAL RESEARCH AND PUBLIC HEALTH, vol. 19, no. 9, 6 May 2022 (2022-05-06), page 5681, XP093044125, DOI: 10.3390/ijerph19095681 * the whole document * ----- | | |

The present search report has been drawn up for all claims

| Place of search | Date of completion of the search | Examiner |
|---|---|---|
| The Hague | 23 May 2023 | Godzina, Przemyslaw |

## ANNEX TO THE EUROPEAN SEARCH REPORT
## ON EUROPEAN PATENT APPLICATION NO.

EP 22 20 9238

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2023

| Patent document cited in search report | Publication date | Patent family member(s) | Publication date |
|---|---|---|---|
| US 2018101664 A1 | 12-04-2018 | CN 104899458 A | 09-09-2015 |
| | | US 2018101664 A1 | 12-04-2018 |
| | | WO 2016201789 A1 | 22-12-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RUSSELL ; BURCH.** Replacement, Reduction and Refinement of animal testing. *The Principles of Humane Experimental Technique,* 1959